# EUROPEAN PATENT SPECIFICATION

(11) **EP 0 705 277 B1**
(45) Date of publication and mention of the grant of the patent: **21.10.1998**
(21) Application number: 94918700.9
(22) Date of filing: 09.06.1994
(51) Int. Cl.: C07K 14/72, C12N 15/12, G01N 33/53, C07K 16/28

(54) **PROSTAGLANDIN RECEPTOR EP3 AND DNA ENCODING IT**
PROSTAGLANDIN REZEPTOR EP3 UND KODIERENDE DNS
RECEPTEUR EP3 DE PROSTAGLANDINES ET ADN CODANT

(30) Priority: 25.06.1993 US 83746
(43) Date of publication of application: 10.04.1996
(73) Proprietor: MERCK FROSST CANADA INC., Kirkland Quebec H9H 3L1 (CA)
(72) Inventor: ABRAMOVITZ, Mark, Dollard des Ormeaux, Quebec H9G 2X8 (CA); BOIE, Yves, Outremont, Quebec H2V 1W5 (CA); METTERS, Kathleen, Montreal, H2X 1W4 (CA); RUSHMORE, Thomas, H., Dollard des Ormeaux, Quebec H9B 2R9 (CA); ADAM, Mohammed, Kirkland, Quebec H9J 2X2 (CA)
(74) Representative: Thompson, John Dr.
(86) International application number: CA9400320
(87) International publication number: WO9500552

(56) References cited:
- EP-A- 0 557 966
- J. BIOL. CHEM. vol. 267, no. 10 , 5 April 1992 pages 6463 - 6466 SUGIMOTO, Y. ET AL. 'Cloning and expression of a cDNA for mouse prostaglandin E receptor EP3 subtype'
- J. BIOL. CHEM. vol. 268, no. 4 , 5 February 1993 pages 2712 - 2718 SUGIMOTO, Y. ET AL. 'Two isoforms of the EP3 receptor with different carboxylterminal domains'
- JPN. J. PHARMACOL., SUPPL. 1 vol. 61 , 1993 page 85P NAMBA, T. ET AL.; SUGIMOTO, Y. ET AL. 'Abstracts O-72, O-73, O-74'

## Description

### BACKGROUND OF THE INVENTION

The physiological actions of prostaglandin (PG)E2 are mediated through interaction with the prostaglandin E receptor(s). There are three subtypes of the EP receptor, EP1, EP2 and EP3 (for review see Coleman et al., 1989 Comprehensive Medicinal Chemistry, 3:643-714). These three subtypes all show high affinity for PGE2 but show differences in their affinities for various agonists and antagonists and exert their actions through different secondary transduction mechanisms. Thus activation of the EP1 receptor is associated with a rise in IP3 and intracellular calcium, activiation of the EP2 receptor results in a rise in intracellular cyclic AMP and activation of the EP3 receptor a fall in intracellular cyclic AMP followed by a rise in intracellular calcium. To date the only members of this family to be cloned are the mouse EP2 (Honda et al., J. Biol. Chem. 1993 266(11):7759-7762) and the mouse EP3a and EP3β (Sugimoto et al., J. Biol. Chem. 1992 267(10):6463-6466; Sugimoto et al., J. Biol. Chem. 1993 268(4):2712-2718) subtypes. EP3 receptors are normally found on a wide variety of cells including the small intestine, kidney, stomach, muscle, eye, uterus and trachea, in humans and other animals. Binding of prostaglandin E2 to the EP3 receptor protein elicits an increase in intracellular calcium levels. This signal causes the tissues to respond, for example by muscle contraction.

The two Sugimoto references diclose different DNA sequences which encode different forms of the EP3 receptor subtype which have substantially different properties with regard to the cytoplasmic domain of the receptor. There is no disclosure or suggestion of the sequence for the human EP3 receptor subtype.

Jpn. J. Pharmacol. Suppl. 1 Vol. 61, 1993, page 85P, Namba et al., abstract 0-72 discloses attempts to isolate EP3 subtypes from a cDNA library generated from a bovine cell line. Again there is no disclosure or suggestion of the sequence for the human EP3 receptor subtype.

### SUMMARY OF THE INVENTION

Novel prostaglandin receptor proteins termed EP3-α, EP3-21 and EP3-9 of the subclass EP3 have been identified from human cells. DNA molecules encoding the full length EP3 proteins have been isolated and purified, and the nucleotide sequences have been determined. The EP3 encoding DNAs have been cloned into expression vectors and these expression vectors, when introduced into recombinant host cells, cause the recombinant host cells to express functional EP3 receptor proteins. The novel EP3 proteins, the EP3-encoding DNAs, the expression vectors and recombinant host cells expressing recombinant EP3 receptors are useful in the identification of modulators of EP3 receptor activity.

A method of identifying EP3 receptor modulators is also disclosed which utilizes the recombinant EP3 expressing host cells. Modulators of EP3 activity are useful for the treatment of prostaglandin-related diseases and for modulating the effects of prostaglandin on the EP3 receptor.

### BRIEF DESCRIPTION OF THE DRAWINGS

Figure 1A-C- - Panel A - The complete DNA sequence encoding the EP3-α receptor protein is shown; Panel B - the complete DNA sequence encoding the EP3-21 receptor protein is shown; and Panel C - the complete DNA sequence encoding the EP3-9 receptor protein is shown.

Figure 2 - Panel A - The complete deduced amino acid sequence of the EP3-α receptor protein is shown; the complete deduced amino acid sequence of the EP3-21 receptor protein is shown; and the complete deduced amino acid sequence of the EP3-9 receptor protein is shown.

Figure 3A-B - Competition for [³H]PGE₂ specific binding to pcDNAIamp-hEP3α transfected COS-M6 membranes is shown in the presence of: Panel A: 0.03 nM-10 µM PGE₂ (Δ), PGE₁ (■), PGF_{2α} (□) and PGD₂ (●); and Panel B: 0.3 nM-100 µM misoprostol (□), AH6809 (Δ) and butaprost (■).

Figure 4A-B - Competition for [³H]PGE₂ specific binding to pcDNAIamp-hEP3-21 transfected COS-M6 membranes is shown in the presence of: Panel A: 0.03 nM-10 µM PGE₂ (Δ), PGE₁ (■), PGF_{2α} (□) and PGD₂ (●); and Panel B: 0.3 nM-100 µM misoprostol (□), AH6809 (Δ) and butaprost (■).

Figure 5A-B - Competition for [³H]PGE₂ specific binding to pcDNA-hEP3-9 transfected COS-M6 membranes is shown in the presence of: Panel A: 0.03 nM-10 µM PGE₂ (Δ), PGE₁ (■), PGF_{2α} (□) and PGD₂ (●); and Panel B: 0.3 nM-100 µM misoprostol (□), AH6809 (A) and butaprost (■).

### DETAILED DESCRIPTION OF THE INVENTION

The present invention relates to cDNA encoding three novel prostaglandin receptor isotypes termed EP3-α, EP3-21 and EP3-9 of the subtype EP3. The present invention is also related to recombinant host cells which express the cloned EP3-encoding DNAs contained in a recombinant expression plasmid. The present invention is also related to a method for the screening of substances which modulate EP3 receptor activity. The DNAs of the present invention are isolated from EP3 producing cells. EP3, as used herein, refers to a G protein-coupled receptor which can specifically bind prostaglandin molecules. The present invention also relates to a unique prostaglandin receptor protein, also described as EP3, which is isolated from EP3, producing cells. EP3 receptor protein, as used herein, refers to a G protein-coupled type receptor which is present in three isotypes and which can specifically bind prostaglandin molecules.

Mammalian cells capable of producing EP3 include, but are not limited to, cells derived from small intestine, kidney, stomach, muscle, eye, uterus and trachea. The preferred cells for the present invention include normal human kidney or uterine cells

Other cells and cell lines may also be suitable for use to isolate EP3 cDNA. Selection of suitable cells may be done by screening for EP3 on cell surfaces. Methods for detecting EP3 activity are well known in the art and measure the binding of radiolabelled ligand specific for the receptor. Cells which possess EP3 activity in this assay may be suitable for the isolation of EP3 cDNA.

Any of a variety of procedures may be used to clone EP3 cDNA. These methods include, but are not limited to, direct functional expression of the EP3 cDNA following the construction of an EP3-containing cDNA library in an appropriate expression vector system. Another method is to screen an EP3-containing cDNA library constructed in a bacteriophage or plasmid shuttle vector with a labelled oligonucleotide probe designed from the amino acid sequence of the EP3 protein. The preferred method consists of screening an EP3-containing cDNA library constructed in a bacteriophage or plasmid shuttle vector with a partial cDNA encoding the EP3 protein. This partial cDNA is obtained by the specific PCR amplification of EP3 DNA fragments through the design of degenerate oligonucleotide primers from the amino acid sequence known for other G protein-coupled receptors which are related to the prostaglandin EP3 receptors.

It is readily apparent to those skilled in the art that other types of libraries, as well as libraries constructed from other cells or cell types, may be useful for isolating EP3-encoding DNA. Other types of libraries include, but are not limited to, cDNA libraries derived from other cells or cell lines other than human kidney cells, and genomic DNA libraries.

It is readily apparent to those skilled in the art that suitable cDNA libraries may be prepared from cells or cell lines which have EP3 activity. The selection of cells or cell lines for use in preparing a cDNA library to isolate EP3 cDNA may be done by first measuring cell associated EP3 activity using the known labelled ligand binding assay cited above and used herein.

Preparation of cDNA libraries can be performed by standard techniques well known in the art. Well known cDNA library construction techniques can be found for example, in Maniatis, T., Fritsch, E.F., Sambrook, J., Molecular Cloning: A Laboratory Manual (Cold Spring Harbor Laboratory, Cold Spring Harbor, New York, 1982).

It is also readily apparent to those skilled in the art that DNA encoding EP3 may also be isolated from a suitable genomic DNA library.

Construction of genomic DNA libraries can be performed by standard techniques well known in the art. Well known genomic DNA library construction techniques can be found in Maniatis, T., Fritsch, E.F., Sambrook, J. in Molecular Cloning: A Laboratory Manuel (Cold Spring Harbor Laboratory, Cold Spring Harbor, New York, 1982).

In order to clone the EP3 gene by one of the preferred methods, the amino acid sequence or DNA sequence of EP3 or a homologous protein is necessary. To accomplish this, EP3 protein or a homologous protein may be purified and partial amino acid sequence determined by automated sequenators. It is not necessary to determine the entire amino acid sequence, but the linear sequence of two regions of 6 to 8 amino acids can be determined for the PCR amplification of a partial EP3 DNA fragment.

Once suitable amino acid sequences have been identified, the DNA sequences capable of encoding them are synthesized. Because the genetic code is degenerate, more than one codon may be used to encode a particular amino acid, and therefore, the amino acid sequence can be encoded by any of a set of similar DNA oligonucleotides. Only one member of the set will be identical to the EP3 sequence but others in the set will be capable of hybridizing to EP3 DNA even in the presence of DNA oligonucleotides with mismatches. The mismatched DNA oligonucleotides may still sufficiently hybridize to the EP3 DNA to permit identification and isolation of EP3 encoding DNA.

Using one of the preferred methods, cDNA clones encoding EP3 are isolated in a two-stage approach employing polymerase chain reaction (PCR) based technology and cDNA library screening. In the first stage, NH2-terminal and internal amino acid sequence information from the purified EP3 or a homologous protein is used to design degenerate oligonucleotide primers for the amplification of EP3-specific DNA fragments. In the second stage, these fragments are cloned to serve as probes for the isolation of full length cDNA from a cDNA library derived from human kidney cells.

The sequence for the three near full-length cDNAs encoding EP3 are shown in Table 1, and were designated clone EP3-α, EP3-21 and EP3-9. The deduced amino acid sequences of EP3 from the three cloned cDNA is shown in Table 2. Inspection of the determined cDNA sequences reveals the presence of single, large open reading frames that encode for a 390, 388 and 365 amino acid proteins respectively.

The cloned EP3 cDNA obtained through the methods described above may be recombinantly expressed by molecular cloning into an expression vector containing a suitable promoter and other appropriate transcription regulatory elements, and transferred into prokaryotic or eukaryotic host cells to produce recombinant EP3. Techniques for such manipulations can be found described in Maniatis, T, et al., supra, and are well known in the art.

Expression vectors are defined herein as DNA sequences that are required for the transcription of cloned DNA and the translation of their mRNAs in an appropriate host. Such vectors can be used to express eukaryotic DNA in a variety of hosts such as bacteria, bluegreen algae, plant cells, insect cells and animal cells.

Specifically designed vectors allow the shuttling of DNA between hosts such as bacteria-yeast or bacteria-animal cells. An appropriately constructed expression vector should contain: an origin of replication for autonomous replication in host cells, selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses.

A variety of mammalian expression vectors may be used to express recombinant EP3 in mammalian cells. Commercially available mammalian expression vectors which may be suitable for recombinant EP3 expression, include but are not limited to, pMClneo (Stratagene), pXT1 (Stratagene), pSG5 (Stratagene), pcDNAI, pcDNAIamp (Invitrogen), EBO-pSV2-neo (ATCC 37593) pBPV-1(8-2) (ATCC 37110), pdBPV-MMTneo(342-12) (ATCC 37224), pRSVgpt (ATCC 37199), pRSVneo (ATCC 37198), pSV2-dhfr (ATCC 37146), pUCTag (ATCC 37460), and lZD35 (ATCC 37565).

DNA encoding EP3 may also be cloned into an expression vector for expression in a host cell. Host cells may be prokaryotic or eukaryotic, including but not limited to bacteria, yeast, mammalian cells including but not limited to cell lines of human, bovine, porcine, monkey and rodent origin, and insect cells including but not limited to drosophila derived cell lines. Cell lines derived from mammalian species which may be suitable and which are commercially available, include but are not limited to, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171).

The expression vector may be introduced into host cells via any one of a number of techniques including but not limited to transformation, transfection, protoplast fusion, and electroporation. The expression vector-containing cells are individually analyzed to determine whether they produce EP3 protein. Identification of EP3 expressing cells may be done by several means, including but not limited to immunological reactivity with anti-EP3 antibodies, and the presence of host cell-associated EP3 activity.

Expression of EP3 DNA may also be performed using in vitro produced synthetic mRNA. Synthetic mRNA can be efficiently translated in various cell-free systems, including but not limited to wheat germ extracts and reticulocyte extracts, as well as efficiently translated in cell based systems, including but not limited to microinjection into frog oocytes, with microinjection into frog oocytes being preferred.

To determine the EP3 cDNA sequence(s) that yields optimal levels of receptor activity and/or EP3 protein, EP3 cDNA molecules including but not limited to the following can be constructed: the full-length open reading frame of the EP3 cDNA and various constructs containing portions of the cDNA encoding only specific domains of the receptor protein or rearranged domains of the protein. All constructs can be designed to contain none, all or portions of the 5' and/or 3' untranslated region of EP3 cDNA. EP3 activity and levels of protein expression can be determined following the introduction, both singly and in combination, of these constructs into appropriate host cells. Following determination of the EP3 cDNA cassette yielding optimal expression in transient assays, this EP3 cDNA construct is transferred to a variety of expression vectors (including recombinant viruses), including but not limited to those for mammalian cells, plant cells, insect cells, oocytes, E. Coli, and yeast cells.

Mammalian cell transfectants are analyzed for both the levels of EP3 receptor activity and levels of EP3 protein by the following methods. Assessing EP3 receptor activity involves the direct introduction of a labelled ligand to the cells and determining the amount of specific binding of the ligand to the EP3-expressing cells. Binding assays for receptor activity are known in the art (Frey et al., 1993, Eur. J. Pharmacol., 244, pp 239-250).

Levels of EP3 protein in host cells is quantitated by a variety of techniques including, but not limited to, immunoaffinity and/or ligand affinity techniques. EP3-specific affinity beads or EP3-specific antibodies are used to isolate ³⁵S-methionine labelled or unlabelled EP3 protein. Labelled EP3 protein is analyzed by SDS-PAGE. Unlabelled EP3 protein is detected by Western blotting, ELISA or RIA assays employing EP3 specific antibodies.

Following expression of EP3 in a host cell, EP3 protein may be recovered to provide EP3 in active form, capable of binding EP3-specific ligands. Several EP3 purification procedures are available and suitable for use. Recombinant EP3 may be purified from cell membranes by various combinations of, or individual application of standard separation techniques including but not limited to detergent solubilization, salt fractionation, ion exchange chromatography, size exclusion chromatography, hydroxylapatite adsorption chromatography and hydrophobic interaction chromatography.

In addition, recombinant EP3 can be separated from other cellular proteins by use of an immuno-affinity column made with monoclonal or polyclonal antibodies specific for full length nascent EP3, or polypeptide fragments of EP3.

Monospecific antibodies to EP3 are purified from mammalian antisera containing antibodies reactive against EP3 or are prepared as monoclonal antibodies reactive with EP3 using the technique of Kohler and Milstein, Nature 256: 495-497 (1975). Monospecific antibody as used herein is defined as a single antibody species or multiple antibody species with homogenous binding characteristics for EP3. Homogenous binding as used herein refers to the ability of the antibody species to bind to a specific antigen or epitope, such as those associated with the EP3, as described above. EP3 specific antibodies are raised by immunizing animals such as mice, rats, guinea pigs, rabbits, goats, horses and the like, with an appropriate concentration of EP3 or peptides derived from the EP3 sequence, either with or without an immune adjuvant.

Preimmune serum is collected prior to the first immunization. Each animal receives between about 0.1 µg and about 1000 µg of EP3 or EP3 related peptides associated with an acceptable immune adjuvant. Such acceptable adjuvants include, but are not limited to, Freund's complete, Freund's incomplete, alum-precipitate, water in oil emulsion containing Corynebacterium parvum and tRNA. The initial immunization consisted of the enzyme in, preferably, Freund's complete adjuvant at multiple sites either subcutaneously (SC), intraperitoneally (IP) or both. Each animal is bled at regular intervals, preferably weekly, to determine antibody titer. The animals may or may not receive booster injections following the initial immunizaiton. Those animals receiving booster injections are generally given an equal amount of EP3 or EP3 related peptide in Freund's incomplete adjuvant by the same route. Booster injections are given at about three week intervals until maximal titers are obtained. At about 7 days after each booster immunization or about weekly after a single immunization, the animals are bled, the serum collected, and aliquots are stored at about -20°C.

Monoclonal antibodies (mAb) reactive with EP3 or peptides derived from the EP3 sequence, are prepared by immunizing inbred mice, preferably Balb/c, with EP3 or peptides derived from the sequence of the EP3 proteins. The mice are immunized by the IP or SC route with about 1 µg to about 100 µg, preferably about 10 µg, of EP3 in about 0.5 ml buffer or saline incorporated in an equal volume of an acceptable adjuvant, as discussed above. Freund's complete adjuvant is preferred. The mice receive an initial immunization on day 0 and are rested for about 3 to about 30 weeks. Immunized mice are given one or more booster immunizations of about 1 to about 100 µg of EP3 in a buffer solution such as phosphate buffered saline by the intravenous (IV) route. Lymphocytes, from antibody positive mice, preferably splenic lymphocytes, are obtained by removing spleens from immunized mice by standard procedures known in the art. Hybridoma cells are produced by mixing the splenic lymphocytes with an appropriate fusion partner, preferably myeloma cells, under conditions which will allow the formation of stable hybridomas. Fusion partners may include, but are not limited to: mouse myelomas P3/NS1/Ag 4-1; MPC-11; S-194 and Sp 2/0, with Sp 2/0 being preferred. The antibody producing cells and myeloma cells are fused in polyethylene glycol, about 1000 mol. wt., at concentrations from about 30% to about 50%. Fused hybridoma cells are selected by growth in hypoxanthine, thymidine and aminopterin supplemented Dulbecco's Modified Eagles Medium (DMEM) by procedures known in the art. Supernatant fluids are collected from growth positive wells on about days 14, 18, and 21 and are screened for antibody production by an immunoassay such as solid phase immunoradioassay (SPIRA) using EP3 or peptides derived from the EP3 sequence, as the antigen. The culture fluids are also tested in the Ouchterlony precipitation assay to determine the isotype of the mAb. Hybridoma cells from antibody positive wells are cloned by a technique such as the soft agar technique of MacPherson, Soft Agar Techniques, in Tissue Culture Methods and Applications, Kruse and Paterson, Eds., Academic Press, 1973.

Monoclonal antibodies are produced in vivo by injection of pristine primed Balb/c mice, approximately 0.5 ml per mouse, with about 2 x 106 to about 6 x 106 hybridoma cells about 4 days after priming. Ascites fluid is collected at approximately 8-12 days after cell transfer and the monoclonal antibodies are purified by techniques known in the art.

In vitro production of anti-EP3 mAb is carried out by growing the hydridoma in DMEM containing about 2% fetal calf serum to obtain sufficient quantities of the specific mAb. The mAb are purified by techniques known in the art.

Antibody titers of ascites or hybridoma culture fluids are determined by various serological or immunological assays which include, but are not limited to, precipitation, passive agglutination, enzyme-linked immunosorbent antibody (ELISA) technique and radioimmunoassay (RIA) techniques. Similar assays are used to detect the presence of EP3 in body fluids or tissue and cell extracts.

It is readily apparent to those skilled in the art that the above described methods for producing monospecific antibodies may be utilized to produce antibodies specific for EP3 polypeptide fragments, or full-length EP3 polypeptide.

EP3 antibody affinity columns are made by adding the antibodies to Affigel-10 (Biorad), a gel support which is pre-activated with N-hydroxysuccinimide esters such that the antibodies form covalent linkages with the agarose gel bead support. The antibodies are then coupled to the gel via amide bonds with the spacer arm. The remaining activated esters are then quenched with 1M ethanolamine HC1 (pH 8). The column is washed with water followed by 0.23 M glycine HCl (pH 2.6) to remove any non-conjugated antibody or extraneous protein. The column is then equilibrated in phosphate buffered saline (pH 7.3) together with appropriate membrane solubilizing agents such as detergents and the cell membrane extracts containing EP3 or EP3 fragments are slowly passed through the column. The column is then washed with phosphate buffered saline together with detergents until the optical density (A₂₈₀) falls to background, then the protein is eluted with 0.23 M glycine-HCl (pH 2.6) together with detergents. The purified EP3 protein is then dialyzed against phosphate buffered saline together with detergents.

One method suitable for the isolation of DNA encoding the prostaglandin receptor of the present invention involves the utilization of amino acid and/or DNA sequence information obtained from other G-protein-linked receptors. Since other prostaglandin receptors are known to be G-protein linked, certain regions or domains such as the transmembrane and/or cytoplasmic domains, are expected to have some degree of homology sufficient to produce a probe for the isolation of novel receptors.

Prostaglandins and leukotrienes are known to transduce their signals via G-protein-linked receptors. Distinct receptors for PGH₂/thromboxane A₂, PGl_{2,} PGE₂, PGD₂, PGF_{2α}, LTB₄, and LTD₄ present in various tissues have been described. Some of the receptors have been solubilized and partially purified (Dutta-Roy, A.K. et al., (1987) JBC, 262, pp. 12685; Tsai, A.L. et al., (1989), JBC, 264, pp 61; 168 - Watawabe, T. et. al., (1990), JBC, 265, pp. 21237) and the human platelet TXA₂ receptor has been purified to apparent homogeneity (Ushikubi, F. et. al., (1989), JBC, 264, pp. 16496). The purified thromboxane receptor exhibited a very broad band on a SDS-polyacrylamide gel centered at ≈ 57 kDa. Enough protein was obtained for partial sequence information.

Oligonucleotide probes were used to screen a human megakaryocytic cell line (MEG-01) cDNA library (Hirata, M. et al., (1991), Nature, 349, pp. 617). A partial length cDNA clone was obtained that, when sequenced, was found to encode the carboxy half of a putative G-protein linked receptor. This clone was then labeled and used to screen a human placenta library. One full-length (≈ 2.9 kb) clone contained extensive 5' and 3' noncoding regions and a 1029 bp open reading frame coding for a 343 amino-acid protein of Mᵣ ≈ 37000. The predicted sequence displays the characteristics of seven transmembrane G-linked receptors including two N-linked glycosylation sites (Asn-4 and Asn-16) in the putative extracellular amino terminal tail (29 residues), conserved Cys residues in extracellular loops 1 and 2 (Cys-105 and Cys-183), and several other conserved residues within transmembrane regions, with the exception of the Asp residue found in transmembrane 3, known to be essential for receptors with small amine-containing ligands ( Strosberg, A.D., (1991), EJB, 196, pp 1). The sequence has a very short predicted third intracellular loop (27 residues). This portion of the molecule could possibly couple to the G-protein (G_{q} or larger G-protein) responsible for interacting with phospholipase C and causing subsequent changes in calcium ion flux (Shenker, A. et al., (1991), JBC, 266, pp. 9309. 173 - Moran, N. et al., (1990), Circulation, Suppl. 82, abstract 1830).

The thromboxane receptor has been expressed in Xenopus oocytes. It can couple with endogenous signal transduction components to elicit a calcium-activated Cl- current recorded by electrophysiological measurement using the procedure described by Hirata, M. et al., (1991), Nature, 349, pp. 617. Binding studies have been performed with COS-1 cell membranes transfected by thromboxane receptor cDNA using the ligand S-145 (Hirata, M. et al., (1991), Nature, 349, pp. 617). We have also shown high affinity binding of the thromboxane antagonist SQ-29548 in human embryonic kidney 293 cells and membranes transfected with thromboxane-receptor cDNA with maximal binding of 2-3 pmol/mg protein. This level of expression is at least 5-10 times higher than in platelet membranes. On a per-cell basis assuming a 10% transfection efficiency, we estimate ≈ 10⁶ binding sites/tranfected cell as compared to ≈ 1300 sites present on a platelet (Hourani, S.M.O, et al., (1991), Pharmacol. Rev., 43, pp. 243).

Northern-blot analysis revealed the presence of a 2.8-kb band in the MEG-01 cell line, placenta, and lung. The mRNA is probably in the low-abundance category, based on the reported long exposure time (12 days) and amount of poly(A)⁺ RNA loaded (20 µg) to see detectable signals.

An approach to the isolation of other eicosanoid receptor genes by homology screening was taken, with the assumption that these receptors are related in primary structure (Sugimoto, Y. et al., (1992), JBC, 267, pp. 6463). Since these receptors are of the G-protein coupled receptor superfamily there are areas of homology which are likely to be found in the transmembrane region and in the cytoplasmic domains. Therefore, various known G-protein linked receptors related to the prostaglandin receptors may be utilized to provide DNA probes to regions of the receptor protein-encoding DNA sought, which is likely to have homology, such as the transmembrane region.

Using both a 0.4-kb and a 0.7 kb mouse EP3α cDNA fragment which together encode all of the murine EP3a open reading frame, a 1.9-kb cDNA clone (EP3), hereinafter designated EP3a, encoding a 390-amino acid receptor was isolated from a human kidney cDNA library. A 16-fold degenerate 27 mer oligonucleotide, based on 9 amino acids in transmembrane domain VII of the mouse EP3 and human TP receptor was used to screen a human uterus cDNA library. Two additional cDNAs were cloned hereafter designated as EP3-9 (1.4kb) and EP3-21 (1.7kb) encoding 365 and 388 amino acid receptor proteins respectively. Like many other G-protein coupled receptors the EP3 receptors share several common features. Firstly, there are 4 potential N-linked glycosylation sites (Asn 18 , Asn 36, Asn 217 and Asn 308) in putative extracellular regions. Secondly, conserved cysteine residues are found in exofacial loops 1 and 2. The EP3 receptor does not contain an aspartic acid residue in transmembrane three which is characteristic of the receptors binding cationic amino-containing ligands, however, it possesses a conserved arginine found in all eicosanoid receptors within transmembrane seven. This region is the most highly conserved among the eicosanoid receptors. The EP3 receptor is most highly related to the human thromboxane receptor.

The novel prostaglandin receptor of the present invention is suitable for use in an assay procedure for the identification of compounds which modulate the receptor activity. Modulating receptor activity, as described herein includes the inhibition or activation of the receptor and also includes directly or indirectly affecting the normal regulation of the receptor activity. Compounds which modulate the receptor activity include agonists, antagonists and compounds which directly or indirectly affect regulation of the receptor activity.

The prostaglandin receptors of the present invention may be obtained from both native and recombinant sources for use in an assay procedure to identify receptor modulators. In general, an assay procedure to identify prostaglandin receptor modulators will contain the prostaglandin receptor of the present invention, and a test compound or sample which contains a putative prostaglandin receptor modulator. The test compounds or samples may be tested directly on, for example, purified receptor protein whether native or recombinant, subcellular fractions of receptor-producing cells whether native or recombinant, and/or whole cells expressing the receptor whether native or recombinant. The test compound or sample may be added to the receptor in the presence or absence of a known labelled or unlabelled receptor ligand. The modulating activity of the test compound or sample may be determined by, for example, analyzing the ability of the test compound or sample to bind to the receptor, activate the receptor, inhibit receptor activity, inhibit or enhance the binding of other compounds to the receptor, modifying receptor regulation, or modifying an intracellular activity.

The identification of modulators of EP3 receptor activity are useful in treating disease states involving the EP3 receptor activity. Other compounds may be useful for stimulating or inhibiting activity of the receptor. Selective agonists of the EP3 receptor maybe of use in the treatment of glaucoma through their ability to lower intraocular pressure and may have utility as agents for treating the side effects associated with the administration of non-steoridal antiinflammatory agents, in particular gastrointestinal side effects. Compounds which antagonise the EP3 receptor could be of use in the treatment of diseases in which activation of the EP3 receptor results in either cellular proliferation, induction of cellular neoplastic transformations or metastatic tumor growth or pathological states where activation of the EP3 receptor results in smooth muscle contraction, such as observed during renal vasoconstriction. The isolation and purification of an EP3-encoding DNA molecule would be useful for establishing the tissue distribution of EP3 receptors for studying changes in EP3 receptor expression in disease states, as well as establishing a process for identifying compounds which modulate EP3 receptor activity.

The following examples are provided for the purpose of illustrating the present invention without, however, limiting the same thereto.

### EXAMPLE 1

### Cloning of the EP_{3α}, EP₃-9 and EP₃-21 receptor cDNAs

Mouse kidney poly A⁺ RNA was reverse transcribed using an RT-PCR kit from Perkin Elmer (Branchburg, N.J.) followed by PCR carried out with two different sets of PCR primers. The first set of primers included a 5'-sense 25mer oligonucleotide [5'-CCACCATGGCTAGCATGTGGGCGCC-3'] (SEQ.ID.NO.: 1) and a 3'-antisense 25mer oligonucleotide [5'-CTCCACGGCCATGGCGCTGGCCACC-3'](SEQ.ID.NO.: 2). This set generated a 398 bp 5' cDNA fragment of the mouse EP_{3α} receptor. The second set of primers included a 5' sense 8-fold degenerate 27mer oligonucleotide [CTGCC(G,C)(G,C)TGCTGGGCGTGGG(C,T)CGCTAC-3'] (SEQ.ID.NO.: 3) and a 3' antisense 16-fold degenerate 27mer oligonucleotide [5'-ATA(A,C)ACCCAGGG(A,G)TCCA(A,G)GATCTG(G,A)TT-3'] (SEQ.ID.NO.: 4) which generated a 468 bp 3' cDNA fragment of the mouse EP_{3α} receptor. These cDNA fragments were ³²P-labeled and used to probe a human kidney cDNA lambda gt11 library (Clontech, Palo Alto, CA) according to standard techniques (Sambrook et al., 1989. Molecular Cloning: A Laboratory Manual, 2nd Ed., Cold Spring Harbor Laboratory, Cold Spring Harbor, N.Y.). From this screening a 1.9 kb full length human EP_{3α} cDNA clone was plaque-purified and DNA was prepared by the plate lysate method (Sambrook et al., 1989. Molecular Cloning: A Laboratory Manual, 2nd Ed., Cold Spring Harbor Laboratory, Cold Spring Harbor, N.Y.).

An antisense 16-fold degenerate 27mer oligonucleotide [5'-ATA(A,C)ACCCAGGG(A,G)TCCA(A,G)GATCTG(G,A)TT-3'] (SEQ.ID.NO.: 5) based on the 9 conserved amino acids (NQILDPWVY) (SEQ.ID.NO.: 6) in transmembrane domain VII was synthesized. The ³²P-labeled oligo probe was used to screen a human uterus lambda gtlO library (Clontech, Palo Alto, CA) using standard techniques (Sambrook et al., 1989. Molecular Cloning: A Laboratory Manual, 2nd Ed., Cold Spring Harbor Laboratory, Cold Spring Harbor, N.Y.). From this screening both a 1.7 kb full length human EP3-21 cDNA clone and a 1.4 kb full length human EP3-9 cDNA clone were plaque-purified and DNA was prepared by the plate lysate method (Sambrook et al., 1989. Molecular Cloning: A Laboratory Manual, 2nd Ed., Cold Spring Harbor Laboratory, Cold Spring Harbor, N.Y.).

### Subcloning and sequencing of cDNA

The three positive clones were digested with EcoRI and found to contain inserts of about size 1.9kb, 1.7kb and 1.4kb which were found to hybridize with a fragment of the mouse EP3 receptor cDNA probe upon Southern blot analysis. The three EcoRI fragments (EP3 clones designated EP_{3α}, EP3-21 and EP3-9) and various restriction fragments were subcloned into pKS vector (Stratagne, La Jolla, CA) for sequencing using a T7 DNA polymerase sequencing kit (Pharmacia). The DNA was sequenced entirely on both strands using the KS or SK primers or primers generated from the determined sequence. The nucleotide sequence of the three EP3 clones are shown in Table 1 A, B and C. The amino acid sequence for the encoded proteins are shown in Table 2 A, B and C. Each DNA clone, when sequenced, was found to contain significant sequence homology to the human thromboxane receptor cDNA and the putative heptahelical arrangement characteristic of G protein-coupled receptors was evident. The open reading frames of EP_{3α} , EP₃-21 and EP₃-9 were 1170bp, 1164bp and 1095bp that encode proteins with predicted relative molecular masses of 43,315, 42,688 and 40,507, respectively. The ATG assigned as the initiator codon matches the Kozak consensus sequence for translation initiation (Kozak, 1989 J. Cell. Biol., 108, pp 229-241). The EP3 cDNA clones, EP_{3α}, EP3-21 and EP3-9, contain 3'-untranslated regions of about 502 bp, 298 bp and 64 bp, respectively.

### EXAMPLE 2

### Construction of expression vectors

The cDNAs encoding EP3-α, EP3-21 and EP3-9 were subcloned into the Hind III site of pcDNA lamp (Invitrogen) respectively and the correct orientation were verified by BamHI digestion and sequencing.

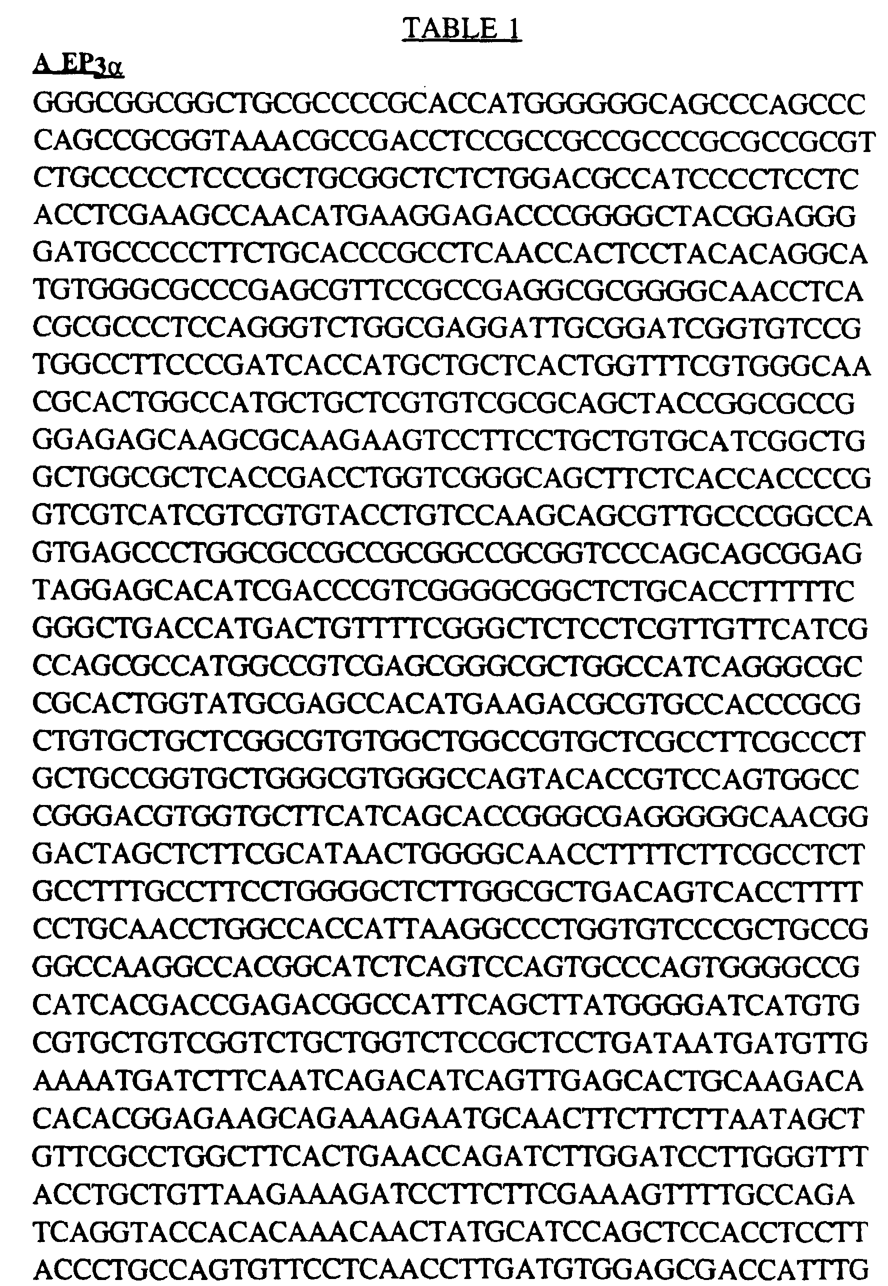

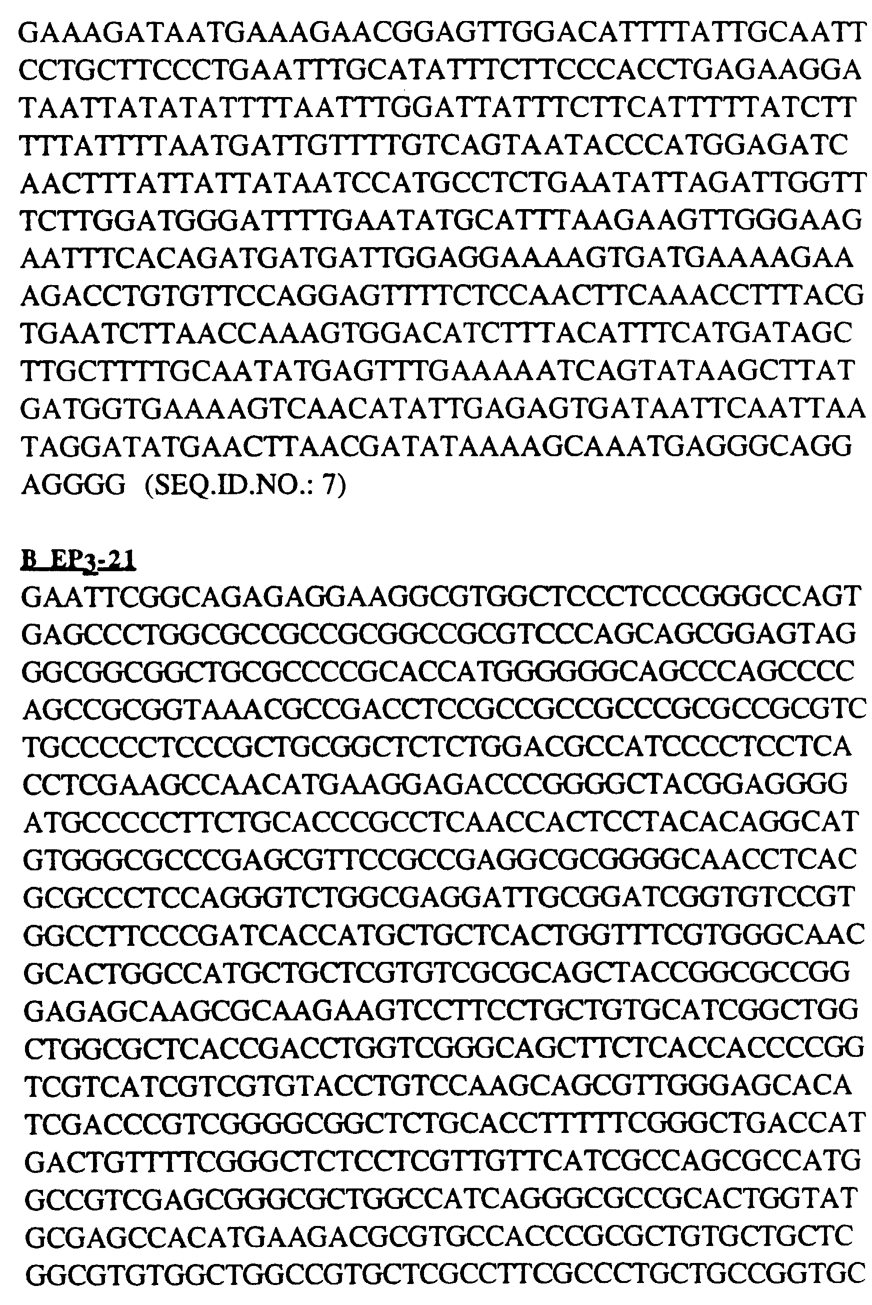

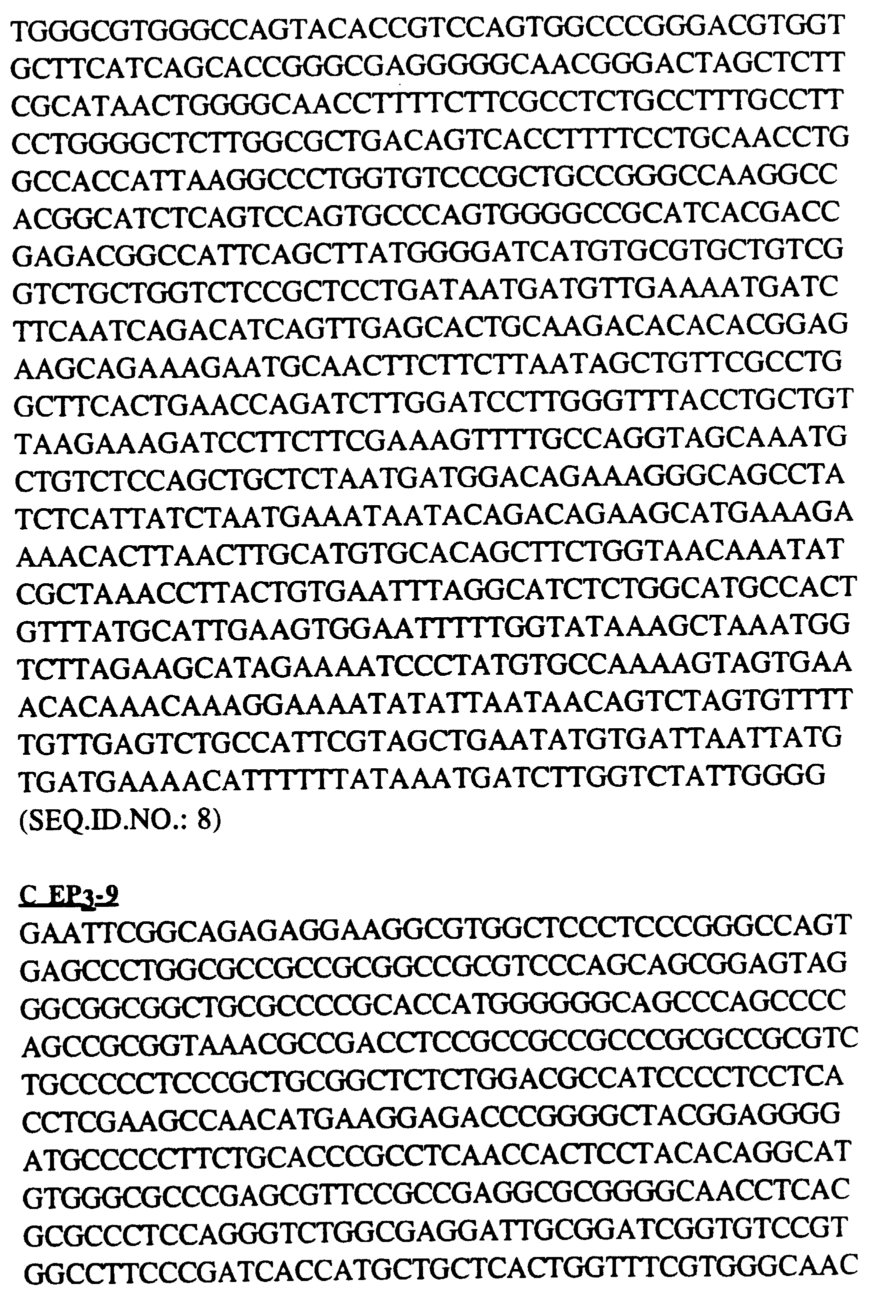

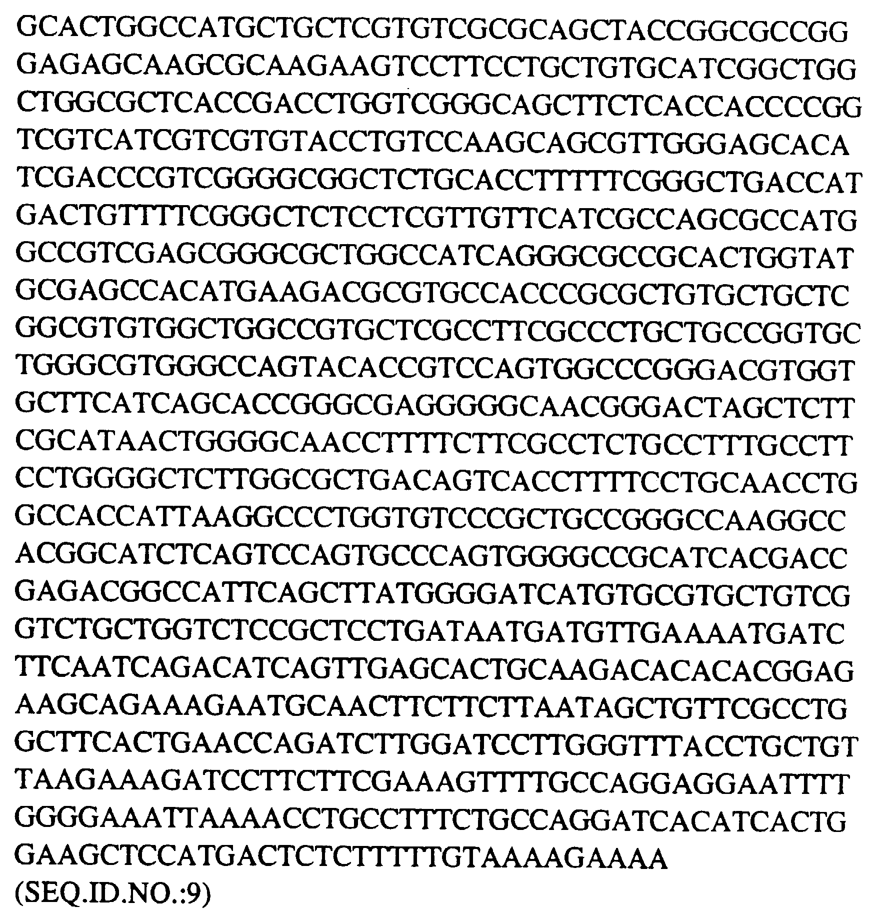

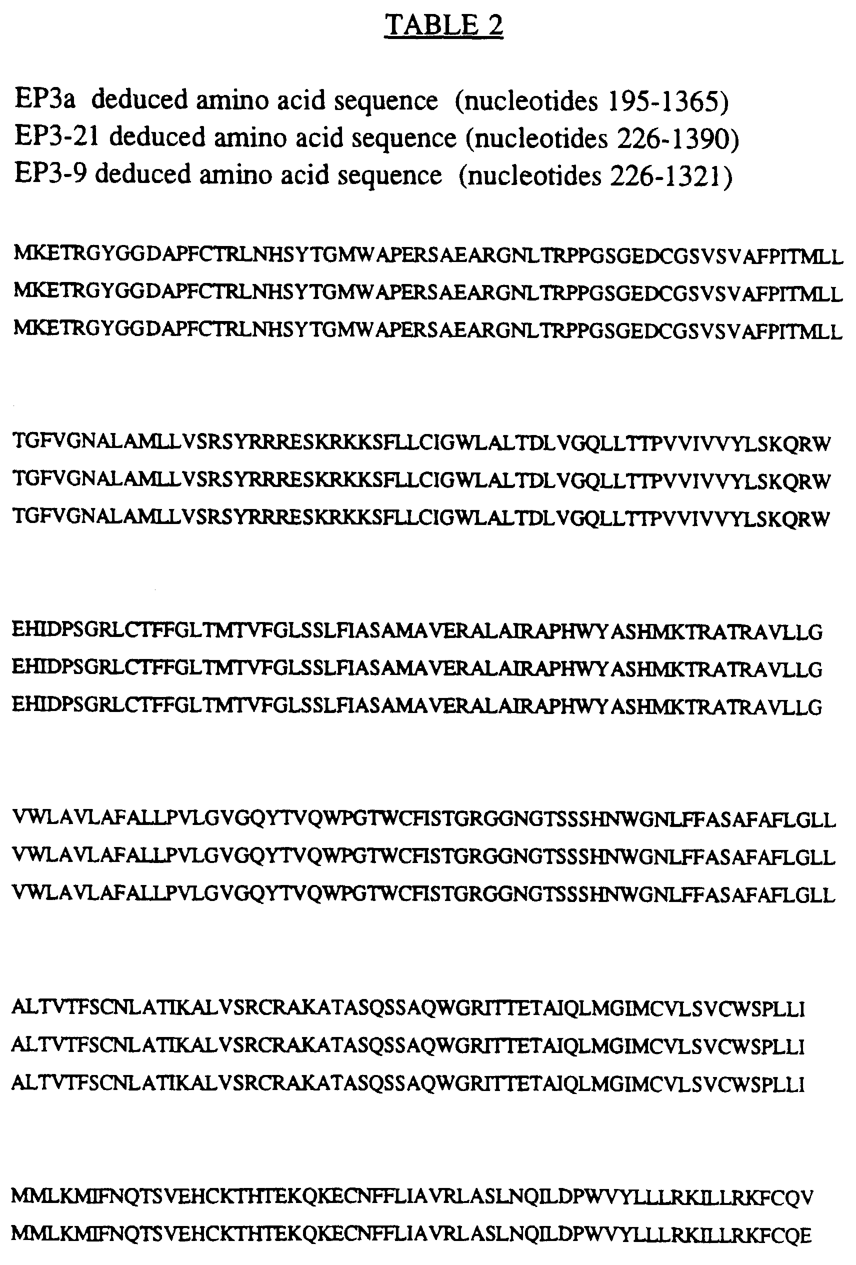

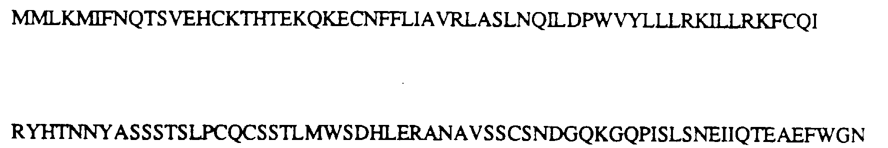

### EXAMPLE 3

### Cloning of the EP3 cDNA into E. coli Expression Vectors

Recombinant EP3 is produced in E. coli following the transfer of the EP3 expression cassette into E. coli expression vectors, including but not limited to, the pET series (Novagen). The pET vectors place EP3 expression under control of the tightly regulated bacteriophage T7 promoter. Following transfer of this construct into an E. coli host which contains a chromosomal copy of the T7 RNA polymerase gene driven by the inducible lac promoter, expression of EP3 is induced when an appropriate lac substrate (IPTG) is added to the culture. The levels of expressed EP3 are determined by the assays described above.

The cDNA encoding the entire open reading frame for EP3 is inserted into the NdeI site of pET 11a. Constructs in the positive orientation are identified by sequence analysis and used to transform the expression host strain BL21. Transformants are then used to inoculate cultures for the production of EP3 protein. Cultures may be grown in M9 or ZB media, whose formulation is known to those skilled in the art. After growth to an approximate OD₆₀₀= 1.5, expression of EP3 is induced with 1 mM IPTG for 3 hours at 37°C. EP3 receptor binding activ ity will be found in membrane fractions from these cells.

### EXAMPLE 4

### In Vitro Translation of EP3 mRNA by Xenopus Oocyte Microinjection and Expression in Mammalian Cells

EP3 cDNA constructs are ligated into in vitro transcription vectors (the pGEM series, Promega) for the production of synthetic mRNAs.

Synthetic mRNA is produced in sufficient quantity in vitro by cloning double stranded DNA encoding EP3 mRNA into a plasmid vector containing a bacteriophage promoter, linearizing the plasmid vector containing the cloned EP3-encoding DNA, and transcribing the cloned DNA in vitro using a DNA-dependent RNA polymerase from a bacteriophage that specifically recognizes the bacteriophage promoter on the plasmid vector.

Various plasmid vectors are available containing a bacteriophage promoter recognized by a bacteriophage DNA-dependent RNA polymerase, including but not limited to plasmids pSP64, pSP65, pSP70, pSP71, pSP72, pSP73, pGEM-3Z, pGEM-4Z, pGEM-3Zf, pGEM-5Zf, pGEM-7Zf, pGEM-9Zf, and pGEM-11Zf, the entire series of plasmids is commercially available from Promega.

The double stranded EP3-encoding DNA is cloned into the bacteriophage promoter containing vector in the proper orientation using one or more of the available restriction endonuclease cloning sites on the vector which are convenient and appropriate for cloning EP3 DNA. The vector with the ligated EP3 DNA is used to transform bacteria, and clonal isolates are analyzed for the presence of the vector with the EP3 DNA in the proper orientation.

Once a vector containing the EP3-encoding DNA in the proper orientation is identified and isolated, it is linearized by cleavage with a restriction endonuclease at a site downstream from, and without disrupting, the EP3 transcription unit. The linearized plasmid is isolated and purified, and used as a template for in vitro transcription of EP3 mRNA.

The template DNA is then mixed with bacteriophage-specific DNA-dependent RNA polymerase in a reaction mixture which allows transcription of the DNA template forming EP3 mRNA. Several bacteriophage-specific DNA-dependent RNA polymerases are available, including but not limited to T3, T7, and SP6 RNA polymerase. The synthetic EP3 mRNA is then isolated and purified.

It may be advantageous to synthesize mRNA containing a 5' terminal cap structure and a 3' poly A tail to improve mRNA stability. A cap structure, or 7-methylguanosine, may be incorporated at the 5'terminus of the mRNA by simply adding 7-methylguanosine to the reaction mixture with the DNA template. The DNA-dependent RNA polymerase incorporates the cap structure at the 5' terminus as it synthesizes the mRNA. The poly A tail is found naturally occurring in many cDNAs but can be added to the 3' terminus of the mRNA by simply inserting a poly A tail-encoding DNA sequence at the 3' end of the DNA template.

The isolated and purified EP3 mRNA is translated using either a cell-free system, including but not limited to rabbit reticulocyte lysate and wheat germ extracts (both commercially available from Promega and New England Nuclear) or in a cell based system, including but not limited to microinjection into Xenopus oocytes, with microinjection into Xenopus oocytes being preferred.

Xenopus oocytes are microinjected with a sufficient amount of synthetic EP3 mRNA to produce EP3 protein. The microinjected oocytes are incubated to allow translation of the EP3 mRNA, forming EP3 protein.

These synthetic mRNAs are injected into Xenopus oocytes (stage 5 -6) by standard procedures [Gurdon, J.B. and Wickens, M.D. Methods in Enzymol. 101: 370-386, (1983)]. Oocytes are harvested and analyzed for EP3 expression as described below.

### EXAMPLE 5

### Cloning of EP3 cDNA into a Mammalian Expression Vector

EP3 cDNA expression cassettes are ligated at appropriate restriction endonuclease sites to the following vectors containing strong, universal mammalian promoters: pBC12BI [Cullen, B.R. Methods in Enzymol. 152: 684-704 1988], and pEE12 (CellTech EP 0 338,841) and its derivatives pSZ9016-1 and p9019. p9019 represents the construction of a mammalian expression vector containing the hCMVIE promoter, polylinker and SV40 polyA element with a selectable marker/amplification system comprised of a mutant gene for dihydrofolate reductase (mDHFR) (Simonsen, C.C. and Levinson, A. D. Proc. Natl. Acad. Sci USA 80: 2495-2499 [1983]) driven by the SV40 early promoter. An SV40 polyadenylation sequence is generated by a PCR reaction defined by primers 13978-120 and 139778-121 using pD5 (Berker and Sharp, Nucl. Acid Res. 13: 841-857 [1985]) as template. The resulting 0.25 Kb PCR product is digested with ClaI and SpeI and ligated into the 6.7 Kb fragment of pEE12 which had been likewise digested. The resultant plasmid is digested with BglII and SfiI to liberate the 3' portion of the SV40 early promoter and the GScDNA from the vector. A 0.73 Kb SfiI-XhoII fragment isolated from plasmid pFR400 (Simonsen, C.C. and Levinson, A. D. Proc. Natl. Acad. Sci USA 80: 2495-2499 [1983]) is ligated to the 5.6 Kb vector described above, reconstituting the SV40 early promoter, and inserting the mdHFR gene. This plasmid is designated p9019. pSZ9016-1 is identical to p9019 except for the substitution of the HIV LTR for the huCMVIE promoter. This vector is constructed by digesting p9019 with XbaI and MluI to remove the huCMVIE promoter. The HIV LTR promoter, from residue -117 to +80 (as found in the vector pCD23 containing the portion of the HIV-1 LTR (Cullen, Cell 46:973 [1986]) is PCR amplified from the plasmid pCD23 using oligonucleotide primers which appended to the ends of the product the MluI and SpeI restriction sites on the 5' side while Hind III and Xba I sites are appended on the 3' side. Following the digestion of the resulting 0.2 kb PCR product with the enzymes MluI and Xba I the fragment is agarose gel-purified and ligated into the 4.3 Kb promoterless DNA fragment to generate the vector pSZ9016-1.

Cassettes containing the EP3 cDNA in the positive orientation with respect to the promoter are ligated into appropriate restriction sites 3' of the promoter and identified by restriction site mapping and/or sequencing. These cDNA expression vectors are introduced into various host cells including, but not limited to: COS-7 (ATCC# CRL1651), CV-1 [Sackevitz et al., Science 238: 1575 (1987)], 293, L cells (ATCC# CRL6362)] by standard methods including but not limited to electroporation,or chemical procedures (cationic liposomes, DEAE dextran, calcium phosphate). Transfected cells and cell culture extracts can be harvested and analyzed for EP3 expression as described below.

All of the vectors used for mammalian transient expression can be used to establish stable cell lines expressing EP3. Unaltered EP3 cDNA constructs cloned into expression vectors will be expected to program host cells to make intracellular EP3 protein. The transfection host cells include, but are not limited to, CV-1 [Sackevitz et al., Science 238: 1575 (1987)], tk-L [Wigler, et al. Cell 11: 223 (1977)], NS/0, and dHFr-CHO [Kaufman and Sharp, J. Mol. Biol. 159: 601, (1982)].

Co-transfection of any vector containing EP3 cDNA with a drug selection plasmid including, but not limited to G418, aminoglycoside phosphotransferase, pLNCX [Miller, A.D. and Rosman G. J. Biotech News 7: 980-990 (1989)]; hygromycin, hygromycin-B phosphotransferase, pLG90 [Gritz. L. and Davies, J., GENE 25: 179 (1983)] ; APRT, xanthine-guanine phosphoribosyl-transferase, pMAM (Clontech) [Murray, et al., Gene 31: 233 (1984)] will allow for the selection of stably transfected clones. Levels of EP3 are quantitated by the assays described above.

EP3 cDNA constructs are ligated into vectors containing amplifiable drug-resistance markers for the production of mammalian cell clones synthesizing the highest possible levels of EP3. Following introduction of these constructs into cells, clones containing the plasmid are selected with the appropriate agent, and isolation of an over-expressing clone with a high copy number of the plasmid is accomplished by selection in increasing concentrations of the agent. The following systems are utilized: the 9016 or the 9019 plasmid containing the mutant DHFR gene [Simonson, C. and Levinson, A., Proc. Natl. Acad. Sci. USA 80: 2495 (1983)], transfected into DHFR-CHO cells and selected in methotrexate; the pEE12 plasmid containing the glutamine synthetase gene, transfected into NS/O cells and selected in methionine sulfoximine (CellTech International Patent Application 2089/10404); and 9016 or other CMV promoter vectors, co-transfected with pDLAT-3 containing the thymidine kinase gene [Colbere and Garopin, F., Proc. Natl. Acad. Sci. 76: 3755 (1979)] in APRT and TK deficient L cells, selected in APRT (0.05 mM azaserine, 0.1 mM adenine, 4 ug/ml adenosine) and amplified with HAT (100 uM hypoxanthine, 0.4 uM aminopterin, 16 uM thymidine).

### EXAMPLE 6

### pcDNAIamp-EP3 expression in COS-M6 cells and [³H]PGE₂ binding assays

The three cloned forms of the human prostaglandin E receptor EP3 subtype (hEP3a, hEP3-21 and hEP3-9) which differ in the carboxyl terminus region of the encoded protein were individually subcloned into the pcDNAlamp plasmid (Invitrogen) and transfected into COS-M6 cells using the DEAE-dextran method. The cells were maintained in culture for 72 hours, then harvested and membranes prepared by differential centrifugation (1000 x g for 10 minutes, then 100,000 x g for 30 minutes) following lysis of the cells by nitrogen cavitation. [³H]Prostaglandin E₂ ([³H]PGE₂) binding assays were performed in 10 mM potassium phosphate pH 6.0, containing 1 mM EDTA, 10 mM MgCl_{2,} 0.5 nM [³H]PGE₂ and 0.5-3 µg of protein from the 100,000 x g membrane fraction. Incubations were conducted for 1 hour at room temperature prior to separation of the bound and free radioligand by rapid filtration as previously described (Frey et al., 1993). Residual [³H]PGE₂ bound to the filter was quantified by liquid scintillation counting. Specific binding was defined as the difference between total binding and non-specific binding, determined in the presence of 1µM PGE₂.

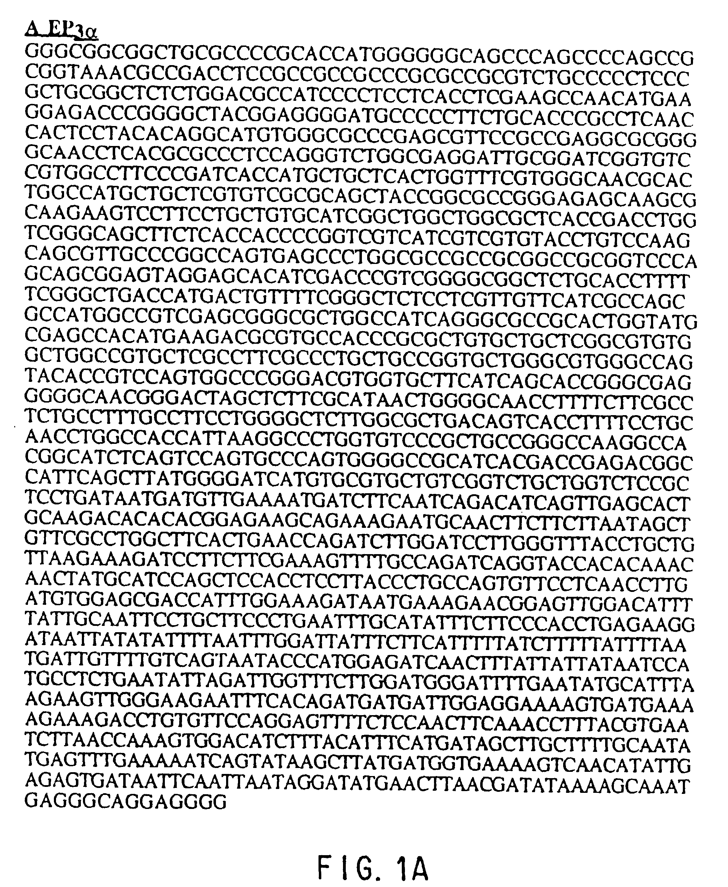

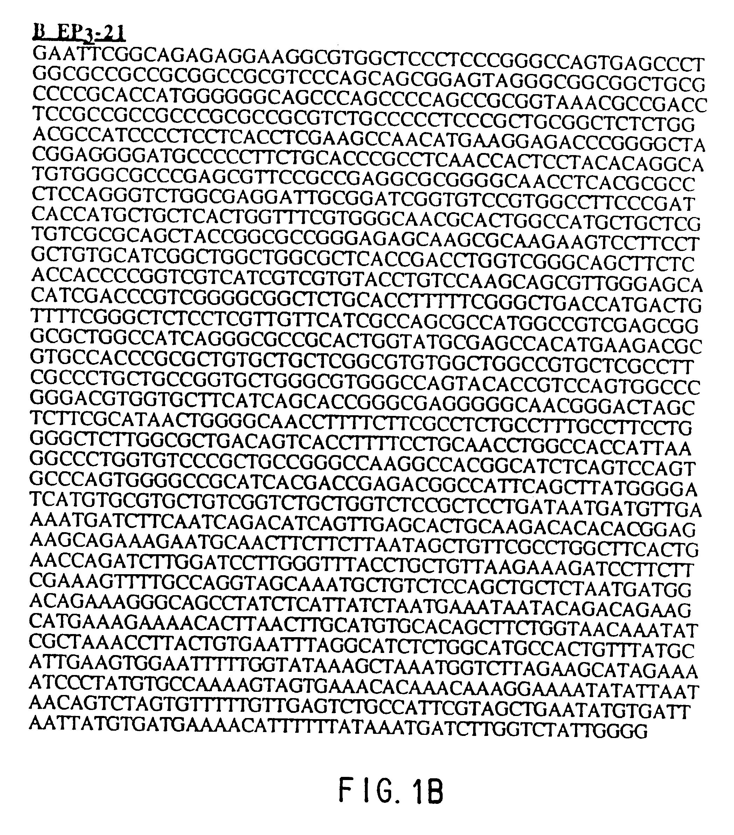

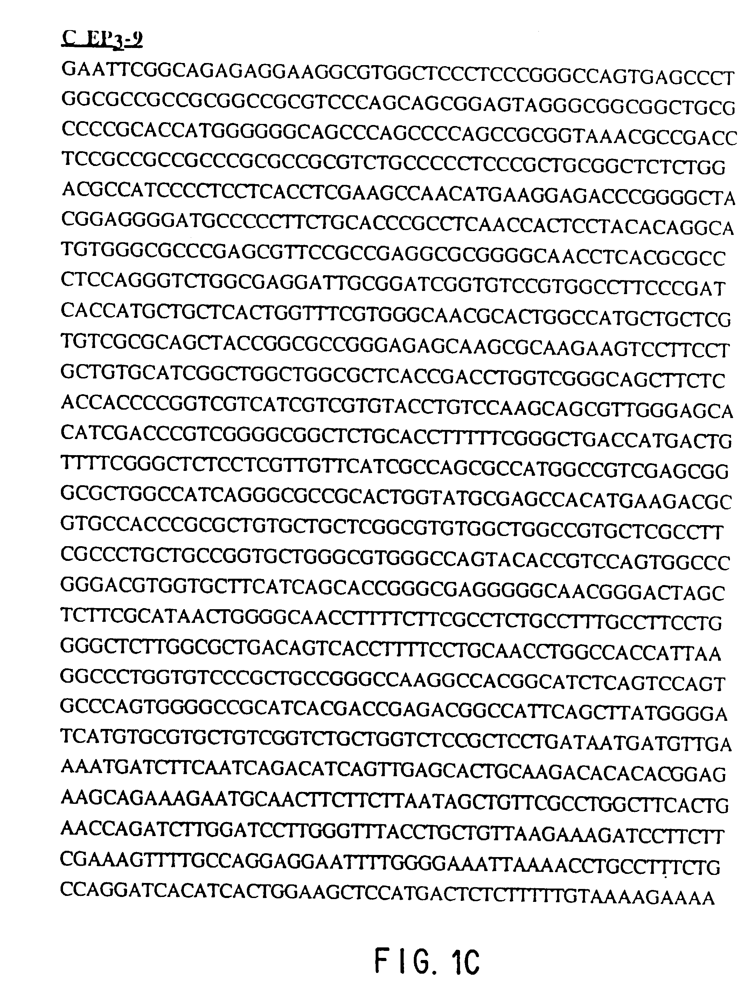

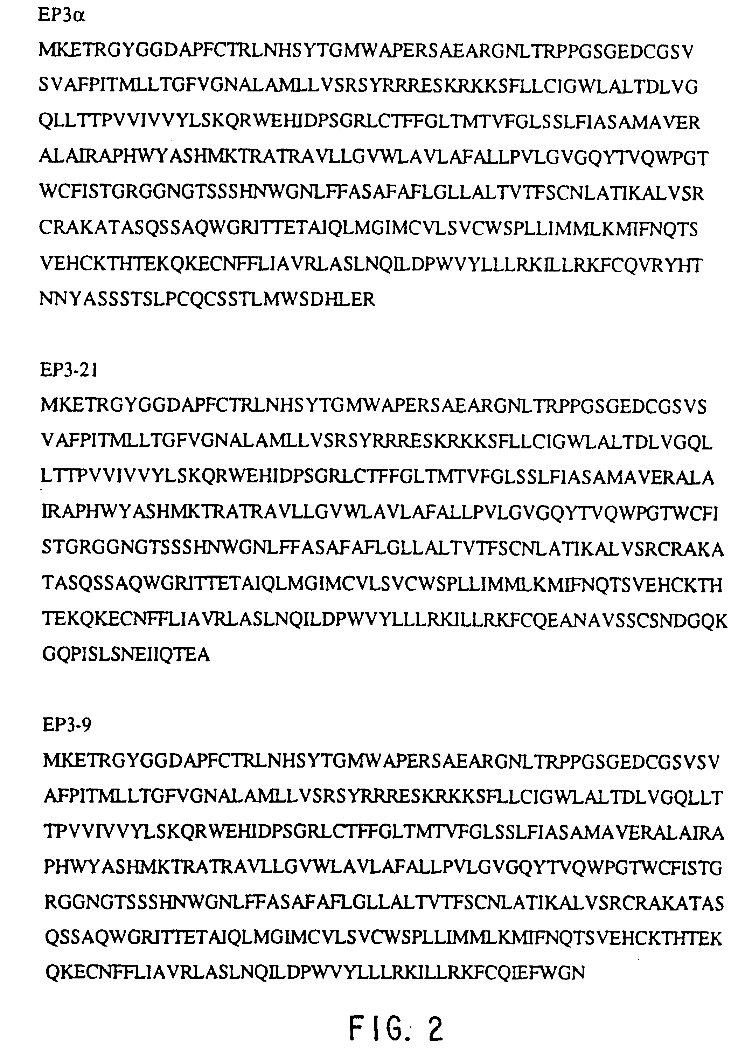

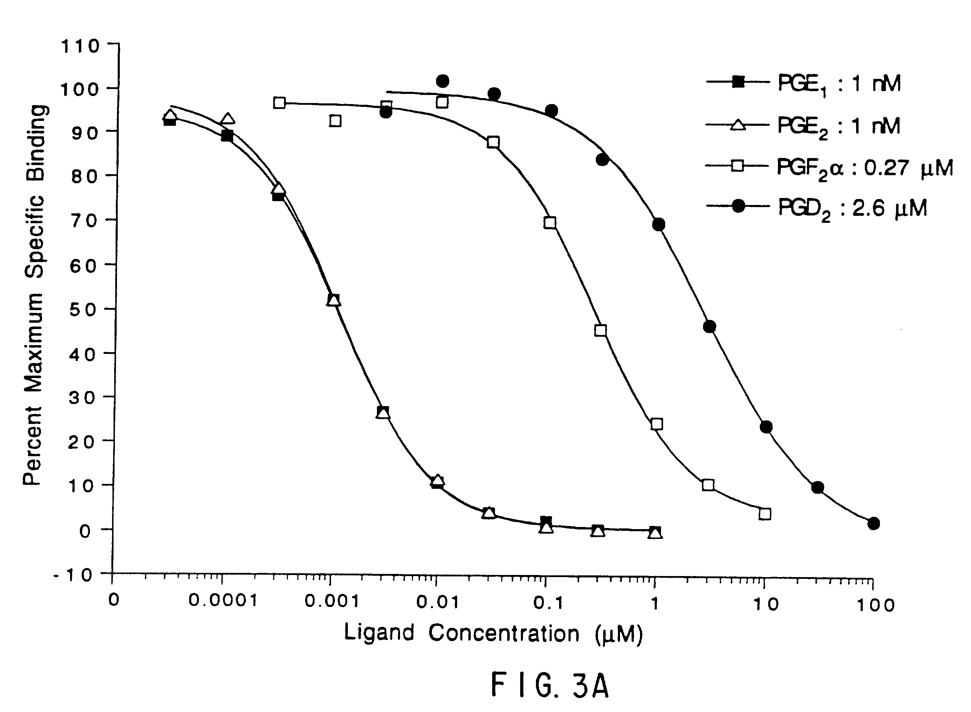

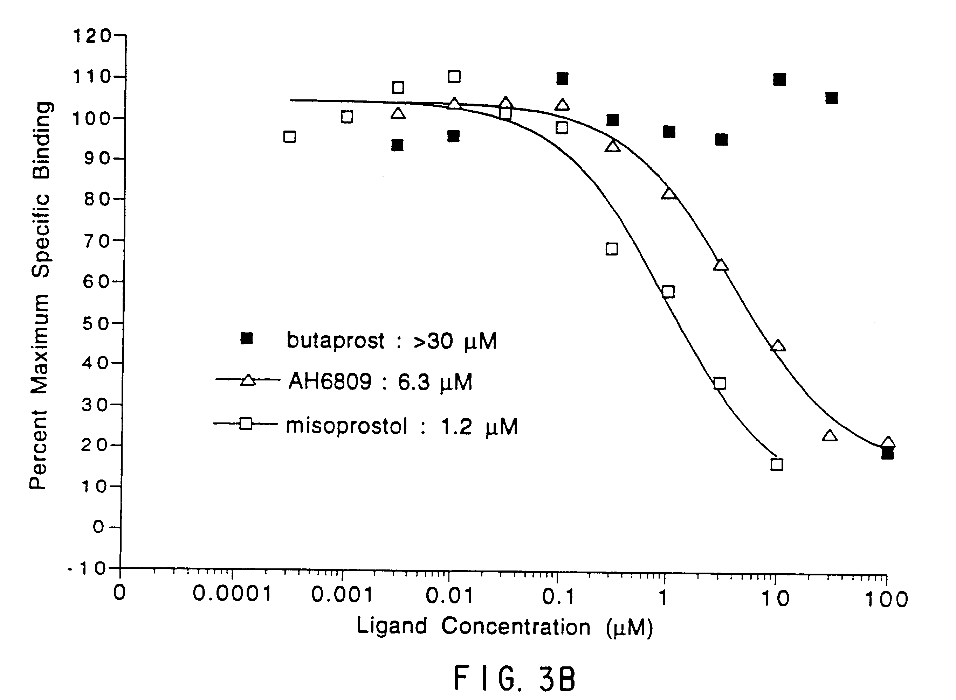

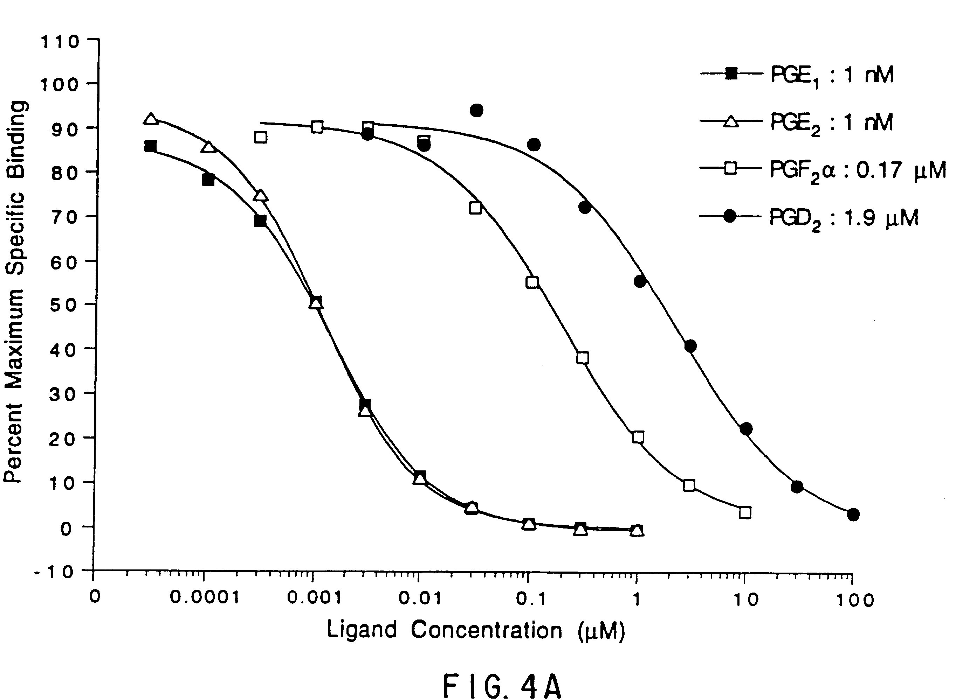

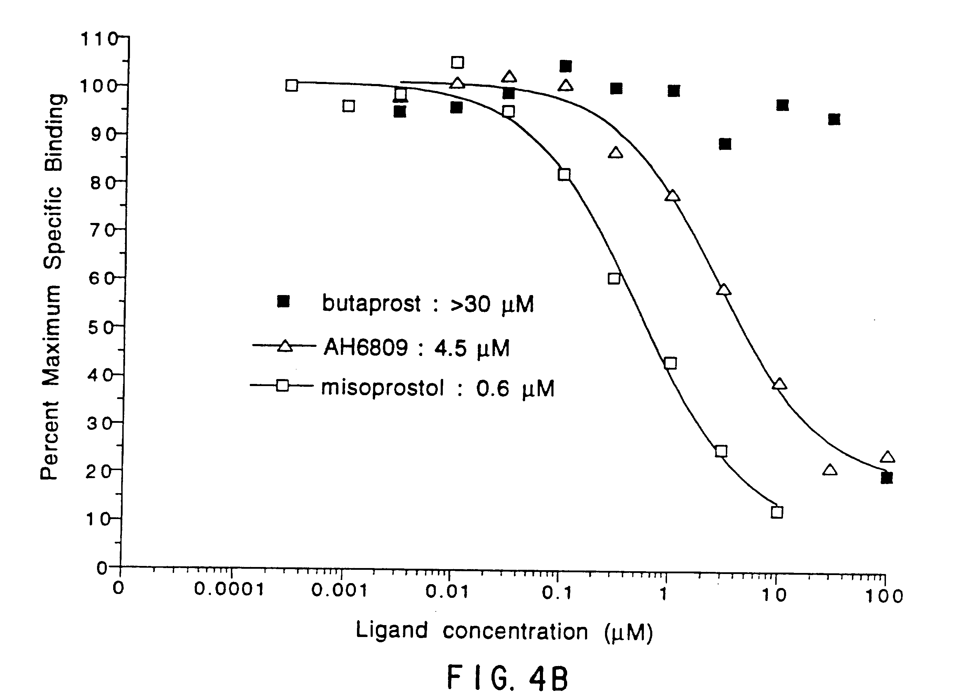

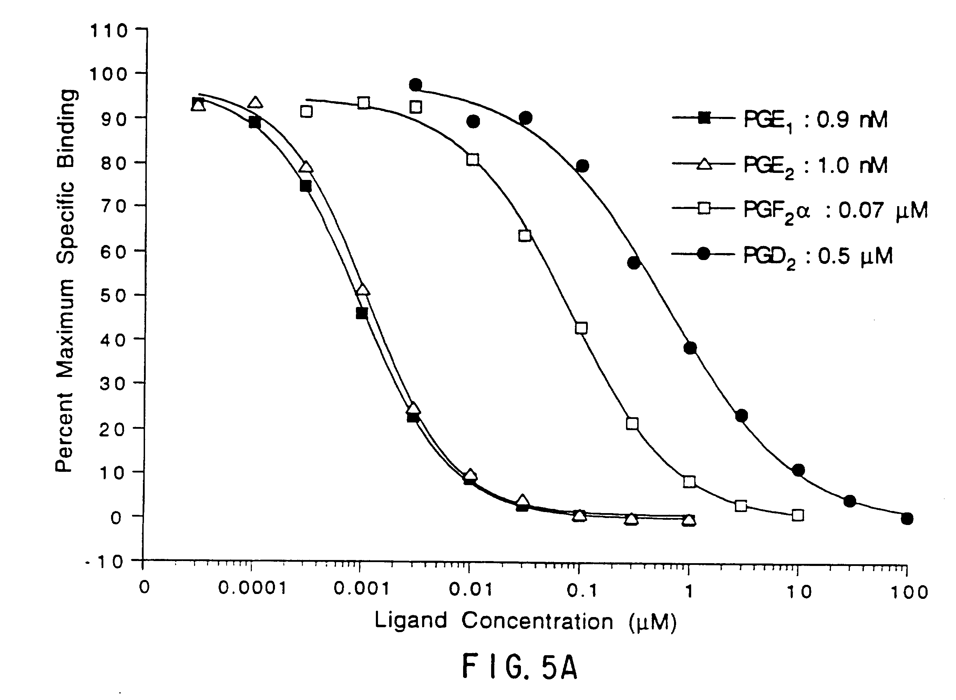

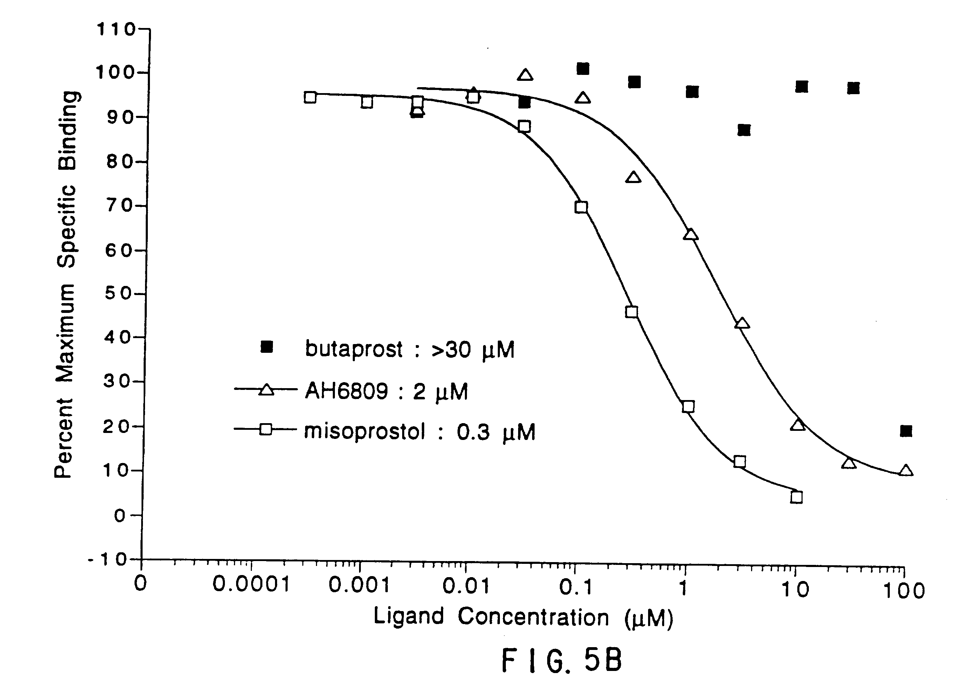

The specific binding of [³H]PGE₂ was of high affinity and saturable in each case. The equilibrium dissociation constant (KD) values were comparable at 0.75 nM, o.83 nM and 0.95 nM for hEP3α, hEP3-21 and hEP3-9, respectively. High expression levels were achieved in all cases with an estimated maximum number of specific [³H]PGE₂ binding sites (Bₘₐₓ) of 13.6 5.3 and 19.8 pmol/mg of membrane protein for hEP3α, hEP3-21 and hEP3-9, respectively. In competition assays PGE₂ and PGE₁ were equipotent in inhibiting [³H]PGE₂ specific binding at all three isoforms with IC50 values of approximately 1 nM (Fig. 3A, 4A and 5A). PGF_{2α} and PGD₂ were approximately 200- and 2000-fold less active than PGE₂ at hEP3α and hEP3-21, but only 65-500-fold less active at hEP3-9 (Fig. 3A, 4A and 5A). Furthermore, the EP1-selective antagonist AH6809 was in general 5-10-fold less active at the hEP3 subtype as compared with the hEP1 subtype with IC50 values ranging from 2 to 7 µM for hEP3 as compared with 0.5 µM for hEP1. In addition, the EP2-selective agonist butaprost was inactive up to 30 µM. Finally, the PGE2 analog misoprostol which is a gastric cytoprotective agent thought to have activity at the EP3 subtype ranged in affinity from 0.3 to 1.2 µM in competition for [³H]PGE₂ specific binding to these isoforms (Fig. 1B, 2B and 3B). These radioligand binding data demonstrate that the hEP3α, hEP3-21 and hEP3-9 isoforms all have the ligand binding characteristics predicted for the EP3 subtype.

### EXAMPLE 7

### Cloning of EP3 cDNA into a Baculovirus Expression Vector for Expression in Insect Cells

Baculovirus vectors, which are derived from the genome of the AcNPV virus, are designed to provide high level expression of cDNA in the Sf9 line of insect cells (ATCC CRL# 1711). Recombinant baculoviruses expressing EP3 cDNA is produced by the following standard methods (In Vitrogen Maxbac Manual): the EP3 cDNA constructs are ligated downstream of the polyhedrin promoter in a variety of baculovirus transfer vectors, including the pAC360 and the pBlueBac vector (In Vitrogen). Recombinant baculoviruses are generated by homologous recombination following co-transfection of the baculovirus transfer vector and linearized AcNPV genomic DNA [Kitts, P.A., Nuc. Acid. Res. 18: 5667 (1990)] into Sf9 cells. Recombinant pAC360 viruses are identified by the absence of inclusion bodies in infected cells (Summers, M. D. and Smith, G. E., Texas Agriculture Exp. Station Bulletin No. 1555) and recombinant pBlueBac viruses are identified on the basis of β-galactosidase expression (Vialard, et al. 1990, J. Virol., 64, pp 37-50). Following plaque purification and infection of sf9 cells with EP3 recombinant baculovirus, EP3 expression is measured by the assays described above.

The cDNA encoding the entire open reading frame for EP3 is inserted into the BamHI site of pBlueBacII. Constructs in the positive orientation with respect to the polyhedrin promoter are identified by sequence analysis and used to transfect Sf9 cells in the presence of linear AcNPV mild type DNA.

Authentic, active EP3 is found associated with the membranes of infected cells. Membrane preparations are prepared from infected cells by standard procedures.

### EXAMPLE 8

### Cloning of EP3 cDNA into a veast expression vector

Recombinant EP3 is produced in the yeast S. cerevisiae following the insertion of the optimal EP3 cDNA construct into expression vectors designed to direct the intracellular expression of heterologous proteins. For intracellular expression, vectors such as EmBLyex4 or the like are ligated to the EP3 cistron [Rinas, U. et al., Biotechnology 8: 543-545 (1990); Horowitz B. et al., J. Biol. Chem. 265: 4189-4192 (1989)]. The levels of expressed EP3 are determined by the assays described above.

### EXAMPLE 9

### Purification of Recombinant EP3

Recombinantly produced EP3 may be purified by antibody affinity chromatography.

EP3 antibody affinity columns are made by adding the anti-EP3 antibodies to Affigel-10 (Biorad), a gel support which is pre-activated with N-hydroxysuccinimide esters such that the antibodies form covalent linkages with the agarose gel bead support. The antibodies are then coupled to the gel via amide bonds with the spacer arm. The remaining activated esters are then quenched with 1M ethanolamine HCI (pH 8). The column is washed with water followed by 0.23 M glycine HCl (pH 2.6) to remove any non-conjugated antibody or extraneous protein. The column is then equilibrated in phosphate buffered saline (pH 7.3) together with appropriate membrane solubilizing agents such as detergents and the cell culture supernatants or cell extracts containing solubilized EP3 or EP3 subunits are slowly passed through the column. The column is then washed with phosphate-buffered saline together with detergents until the optical density (Abs 280) falls to background, then the protein is eluted with 0.23 M glycine-HCl (pH 2.6) together with detergents. The purified EP3 protein is then dialyzed against phosphate buffered saline together with detergents.

## Claims

1. An isolated and purified human prostaglandin EP3 receptor wherein said receptor specifically binds prostaglandin.

2. The isolated and purified prostaglandin receptor protein of Claim 1 wherein said protein is characterized by an amino acid sequence selected from the group consisting of: and

3. An isolated and purified DNA molecule encoding an EP3 prostaglandin receptor protein wherein said protein is characterized by the amino acid sequences of Claim 2.

4. An isolated and purified DNA molecule encoding a prostaglandin receptor protein wherein said DNA molecule is characterized by the nucleotide sequence selected from the group consisting of: and

5. An expression vector for the expression of a prostaglandin EP3 receptor protein in a recombinant host cell wherein said expression vector contains the DNA molecule of Claim 4.

6. A host cell which expresses a recombinant prostaglandin EP3 receptor protein wherein said host cell contains the expression vector of Claim 5.

7. A process for the expression of a prostaglandin EP3 receptor protein in a recombinant host cell, comprising:
(a) transferring the expression vector of Claim 5 into a suitable host cell; and
(b) culturing the host cells under conditions which allow expression of the prostaglandin receptor protein from the expression vectors.

8. A method of identifying modulators of a prostaglandin EP3 receptor activity, comprising:
(a) combining a modulator of prostaglandin receptor activity with the prostaglandin receptor wherein said receptor is characterized by the amino acid sequence of Claim 2 in whole or in part; and
(b) measuring the effect of a modulator on the prostaglandin receptor.

9. An antibody which specifically binds to prostaglandin EP3 receptor protein wherein said protein is characterized by the amino acid sequence of Claim 2.

## Patentansprüche

1. Isolierter und gereinigter Humanprostaglandin-EP3-Rezeptor, wobei der Rezeptor spezifisch Prostaglandin bindet.

2. Isoliertes und gereinigtes Prostaglandin-Rezeptorprotein nach Anspruch 1, wobei das Protein gekennzeichnet ist durch eine Aminosäuresequenz, die ausgewählt ist aus der Gruppe, bestehend aus: und

3. Isoliertes und gereinigtes DNA-Molekül, das für ein EP3-Prostaglandin-Rezeptorprotein kodiert, wobei das Protein durch die Aminosäuresequenzen von Anspruch 2 gekennzeichnet ist.

4. Isoliertes und gereinigtes DNA-Molekül, das für ein Prostaglandin-Rezeptorprotein kodiert, wobei das DNA-Molekül gekennzeichnet ist durch die Nukleotidsequenz, welche ausgewählt ist aus der Gruppe, bestehend aus: und

5. Expressionsvektor für die Expression eines Prostaglandin-EP3-Rezeptorproteins in einer rekombinanten Wirtszelle, wobei der Expressionsvektor das DNA-Molekül nach Anspruch 4 enthält.

6. Wirtszelle, die ein rekombinantes Prostaglandin-EP3-Rezeptorprotein exprimiert, wobei die Wirtszelle den Expressionsvektor nach Anspruch 5 enthält.

7. Verfahren zur Expression eines Prostaglandin-EP3-Rezeptorproteins in einer rekombinanten Wirtszelle, welches umfaßt:
(a) Überführung des Expressionsvektors nach Anspruch 5 in eine geeignete Wirtszelle; und
(b) Kultivierung der Wirtszellen unter Bedingungen, welche die Expression des Prostaglandin-Rezeptorproteins aus den Expressionsvektoren erlauben.

8. Verfahren zur Identifizierung von Modulatoren einer Prostaglandin-EP3-Rezeptoraktivität, welches umfaßt:
(a) Kombination eines Modulators von Prostaglandin-Rezeptoraktivität mit dem Prostaglandin-Rezeptor, wobei der Rezeptor durch die vollständige oder partielle Aminosäuresequenz von Anspruch 2 gekennzeichnet ist; und
(b) Messung der Wirkung eines Modulators auf den Prostaglandin-Rezeptor.

9. Antikörper, der spezifisch an ein Prostaglandin-EP3-Rezeptorprotein bindet, wobei das Protein durch die Aminosäuresequenz von Anspruch 2 gekennzeichnet ist.

## Revendications

1. Récepteur EP3 de prostaglandine humaine isolé et purifié, caractérisé en ce que ledit récepteur fixe spécifiquement la prostaglandine.

2. Protéine de récepteur de prostaglandine isolé et purifié selon la revendication 1, ladite protéine étan caractérisée par une séquence d'aminoacides choisie dans l'ensemble constitué par: et

3. Molécule d'ADN isolée et purifiée codant pour une protéine de récepteur de prostaglandine EP3, ladite protéine étant caractérisée par les séquences d'aminoacides de la revendication 2.

4. Molécule d'ADN isolée et purifiée codant pour une protéine de récepteur de prostaglandine, ladite molécule d'ADN étant caractérisée par la séquence nucléotidique choisie dans l'ensemble constitué par: et

5. Vecteur d'expression pour l'expression d'une protéine de récepteur EP3 de prostaglandine dans une cellule hôte recombinante, caractérisé en ce que ledit vecteur d'expression contient la molécule d'ADN de la revendication 4.

6. Cellule hôte exprimant une protéine de récepteur EP3 de prostaglandine recombinante, caractérisée en ce que ladite cellule hôte contient le vecteur d'expression de la revendication 5.

7. Procédé pour l'expression d'une protéine de récepteur EP3 de prostaglandine dans une cellule hôte recombinante, comprenant:
(a) le transfert du vecteur d'expression de la revendication 5 dans une cellule hôte appropriée; et
(b) la culture des cellules hôtes dans des conditions permettant l'expression de la protéine de récepteur de prostaglandine à partir des vecteurs d'expression.

8. Procédé d'identification de modulateurs d'une activité de récepteur EP3 de prostaglandine, comprenant:
(a) l'association d'un modulateur d'activité de récepteur de prostaglandine avec le récepteur de prostaglandine, ledit récepteur étant caractérisé par la séquence d'aminoacides de la revendication 1 en partie ou en totalité; et
(b) la mesure de l'effet d'un modulateur sur le récepteur de prostaglandine.

9. Anticorps se fixant spécifiquement à la protéine de récepteur EP3 de prostaglandine, ladite protéine étant caractérisée par la séquence d'aminoacides de la revendication 2.


### SEQUENCE LISTING

(1) GENERAL INFORMATION:
   (i) APPLICANT: Abramovitz, Mark
      Boie, Yves
      Metters, Kathleen
      Rushmore, Thomas K
      Adam, Mohammad
   (ii) TITLE OF INVENTION: DNA Encoding Prostaglandin Receptor EP3
   (iii) NUMBER OF SEQUENCES: 12
   (iv) CORRESPONDENCE ADDRESS:
      (A) ADDRESSEE: Merck & Co., Inc.
      (B) STREET: P.O. Box 2000
      (C) CITY: Rahway
      (D) STATE: NJ
      (E) COUNTRY: USA
      (F) ZIP: 07065
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US 08/083,746
      (B) FILING DATE: 25-JUN-1993
      (C) CLASSIFICATION:
   (viii) ATTORNEY/AGENT INFORMATION:
      (A) NAME: Wallen, III, John W.
      (B) REGISTRATION NUMBER: 35,403
      (C) REFERENCE/DOCKET NUMBER: 19026
   (ix) TELECOMMUNICATION INFORMATION:
      (A) TELEPHONE: (908) 594-3905
      (B) TELEFAX: (908) 594-4720
(2) INFORMATION FOR SEQ ID NO:1:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
      (xi) SEQUENCE DESCRIPTION: SEQ ID NO:1:
   CCACCATGGC TAGCATGTGG GCGCC 25
(2) INFORMATION FOR SEQ ID NO:2:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:2:
   CTCCACGGCC ATGGCGCTGG CCACC 25
(2) INFORMATION FOR SEQ ID NO:3:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:3:
   CTGCCSSTGC TGGGCGTGGG YCGCTAC 27
(2) INFORMATION FOR SEQ ID NO:4:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
   ATAMACCCAG GGRTCCARGA TCTGRTT 27
(2) INFORMATION FOR SEQ ID NO:5:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
   ATAMACCCAG GGRTCCARGA TCTGRTT 27
(2) INFORMATION FOR SEQ ID NO:6:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:6:
   Asn Gln Ile Leu Asp Pro Trp Val Tyr
   1               5
(2) INFORMATION FOR SEQ ID NO:7:
   (i) SEQUENCE CHARACTERISTICS:
      (A) LENGTH: 1869 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:7:
   GGGCGGCGGC TGCGCCCCGC ACCATGGGGG GCAGCCCAGC CCCAGCCGCG GTAAACGCCG 60
   ACCTCCGCCG CCGCCCGCGC CGCGTCTGCC CCCTCCCGCT GCGGCTCTCT GGACGCCATC 120
   CCCTCCTCAC CTCGAAGCCA ACATGAAGGA GACCCGGGGC TACGGAGGGG ATGCCCCCTT 180
   CTGCACCCGC CTCAACCACT CCTACACAGG CATGTGGGCG CCCGAGCGTT CCGCCGAGGC 240
   GCGGGGCAAC CTCACGCGCC CTCCAGGGTC TGGCGAGGAT TGCGGATCGG TGTCCGTGGC 300
   CTTCCCGATC ACCATGCTGC TCACTGGTTT CGTGGGCAAC GCACTGGCCA TGCTGCTCGT     360
   GTCGCGCAGC TACCGGCGCC GGGAGAGCAA GCGCAAGAAG TCCTTCCTGC TGTGCATCGG     420
   CTGGCTGGCG CTCACCGACC TGGTCGGGCA GCTTCTCACC ACCCCGGTCG TCATCGTCGT     480
   GTACCTGTCC AAGCAGCGTT GCCCGGCCAG TGAGCCCTGG CGCCGCCGCG GCCGCGGTCC     540
   CAGCAGCGGA GTAGGAGCAC ATCGACCCGT CGGGGCGGCT CTGCACCTTT TTCGGGCTGA     600
   CCATGACTGT TTTCGGGCTC TCCTCGTTGT TCATCGCCAG CGCCATGGCC GTCGAGCGGG     660
   CGCTGGCCAT CAGGGCGCCG CACTGGTATG CGAGCCACAT GAAGACGCGT GCCACCCGCG     720
   CTGTGCTGCT CGGCGTGTGG CTGGCCGTGC TCGCCTTCGC CCTGCTGCCG GTGCTGGGCG     780
   TGGGCCAGTA CACCGTCCAG TGGCCCGGGA CGTGGTGCTT CATCAGCACC GGGCGAGGGG     840
   GCAACGGGAC TAGCTCTTCG CATAACTGGG GCAACCTTTT CTTCGCCTCT GCCTTTGCCT     900
   TCCTGGGGCT CTTGGCGCTG ACAGTCACCT TTTCCTGCAA CCTGGCCACC ATTAAGGCCC     960
   TGGTGTCCCG CTGCCGGGCC AAGGCCACGG CATCTCAGTC CAGTGCCCAG TGGGGCCGCA    1020
   TCACGACCGA GACGGCCATT CAGCTTATGG GGATCATGTG CGTGCTGTCG GTCTGCTGGT    1080
   CTCCGCTCCT GATAATGATG TTGAAAATGA TCTTCAATCA GACATCAGTT GAGCACTGCA    1140
   AGACACACAC GGAGAAGCAG AAAGAATGCA ACTTCTTCTT AATAGCTGTT CGCCTGGCTT    1200
   CACTGAACCA GATCTTGGAT CCTTGGGTTT ACCTGCTGTT AAGAAAGATC CTTCTTCGAA    1260
   AGTTTTGCCA GATCAGGTAC CACACAAACA ACTATGCATC CAGCTCCACC TCCTTACCCT    1320
   GCCAGTGTTC CTCAACCTTG ATGTGGAGCG ACCATTTGGA AAGATAATGA AAGAACGGAG    1380
   TTGGACATTT TATTGCAATT CCTGCTTCCC TGAATTTGCA TATTTCTTCC CACCTGAGAA    1440
   GGATAATTAT ATATTTTAAT TTGGATTATT TCTTCATTTT TATCTTTTTA TTTTAATGAT    1500
   TGTTTTGTCA GTAATACCCA TGGAGATCAA CTTTATTATT ATAATCCATG CCTCTGAATA    1560
   TTAGATTGGT TTCTTGGATG GGATTTTGAA TATGCATTTA AGAAGTTGGG AAGAATTTCA    1620
   CAGATGATGA TTGGAGGAAA AGTGATGAAA AGAAAGACCT GTGTTCCAGG AGTTTTCTCC    1680
   AACTTCAAAC CTTTACGTGA ATCTTAACCA AAGTGGACAT CTTTACATTT CATGATAGCT    1740
   TGCTTTTGCA ATATGAGTTT GAAAAATCAG TATAAGCTTA TGATGGTGAA AAGTCAACAT    1800
   ATTGAGAGTG ATAATTCAAT TAATAGGATA TGAACTTAAC GATATAAAAG CAAATGAGGG    1860
   CAGGAGGGG                                                            1869
(2) INFORMATION FOR SEQ ID NO:8:
   (i) SEQUENCE CHARACTERISTICS:
      (A) LENGTH: 1690 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:8:
   GAATTCGGCA GAGAGGAAGG CGTGGCTCCC TCCCGGGCCA GTGAGCCCTG GCGCCGCCGC     60
   GGCCGCGTCC CAGCAGCGGA GTAGGGCGGC GGCTGCGCCC CGCACCATGG GGGGCAGCCC    120
   AGCCCCAGCC GCGGTAAACG CCGACCTCCG CCGCCGCCCG CGCCGCGTCT GCCCCCTCCC    180
   GCTGCGGCTC TCTGGACGCC ATCCCCTCCT CACCTCGAAG CCAACATGAA GGAGACCCGG    240
   GGCTACGGAG GGGATGCCCC CTTCTGCACC CGCCTCAACC ACTCCTACAC AGGCATGTGG    300
   GCGCCCGAGC GTTCCGCCGA GGCGCGGGGC AACCTCACGC GCCCTCCAGG GTCTGGCGAG    360
   GATTGCGGAT CGGTGTCCGT GGCCTTCCCG ATCACCATGC TGCTCACTGG TTTCGTGGGC    420
   AACGCACTGG CCATGCTGCT CGTGTCGCGC AGCTACCGGC GCCGGGAGAG CAAGCGCAAG    480
   AAGTCCTTCC TGCTGTGCAT CGGCTGGCTG GCGCTCACCG ACCTGGTCGG GCAGCTTCTC    540
   ACCACCCCGG TCGTCATCGT CGTGTACCTG TCCAAGCAGC GTTGGGAGCA CATCGACCCG    600
   TCGGGGCGGC TCTGCACCTT TTTCGGGCTG ACCATGACTG TTTTCGGGCT CTCCTCGTTG    660
   TTCATCGCCA GCGCCATGGC CGTCGAGCGG GCGCTGGCCA TCAGGGCGCC GCACTGGTAT    720
   GCGAGCCACA TGAAGACGCG TGCCACCCGC GCTGTGCTGC TCGGCGTGTG GCTGGCCGTG    780
   CTCGCCTTCG CCCTGCTGCC GGTGCTGGGC GTGGGCCAGT ACACCGTCCA GTGGCCCGGG    840
   ACGTGGTGCT TCATCAGCAC CGGGCGAGGG GGCAACGGGA CTAGCTCTTC GCATAACTGG    900
   GGCAACCTTT TCTTCGCCTC TGCCTTTGCC TTCCTGGGGC TCTTGGCGCT GACAGTCACC    960
   TTTTCCTGCA ACCTGGCCAC CATTAAGGCC CTGGTGTCCC GCTGCCGGGC CAAGGCCACG   1020
   GCATCTCAGT CCAGTGCCCA GTGGGGCCGC ATCACGACCG AGACGGCCAT TCAGCTTATG   1080
   GGGATCATGT GCGTGCTGTC GGTCTGCTGG TCTCCGCTCC TGATAATGAT GTTGAAAATG   1140
   ATCTTCAATC AGACATCAGT TGAGCACTGC AAGACACACA CGGAGAAGCA GAAAGAATGC   1200
   AACTTCTTCT TAATAGCTGT TCGCCTGGCT TCACTGAACC AGATCTTGGA TCCTTGGGTT   1260
   TACCTGCTGT TAAGAAAGAT CCTTCTTCGA AAGTTTTGCC AGGTAGCAAA TGCTGTCTCC 1320
   AGCTGCTCTA ATGATGGACA GAAAGGGCAG CCTATCTCAT TATCTAATGA AATAATACAG 1380
   ACAGAAGCAT GAAAGAAAAC ACTTAACTTG CATGTGCACA GCTTCTGGTA ACAAATATCG 1440
   CTAAACCTTA CTGTGAATTT AGGCATCTCT GGCATGCCAC TGTTTATGCA TTGAAGTGGA 1500
   ATTTTTGGTA TAAAGCTAAA TGGTCTTAGA AGCATAGAAA ATCCCTATGT GCCAAAAGTA 1560
   GTGAAACACA AACAAAGGAA AATATATTAA TAACAGTCTA GTGTTTTTGT TGAGTCTGCC 1620
   ATTCGTAGCT GAATATGTGA TTAATTATGT GATGAAAACA TTTTTTATAA ATGATCTTGG 1680
   TCTATTGGGG 1690
(2) INFORMATION FOR SEQ ID NO:9:
   (i) SEQUENCE CHARACTERISTICS:
      (A) LENGTH: 1387 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
   GAATTCGGCA GAGAGGAAGG CGTGGCTCCC TCCCGGGCCA GTGAGCCCTG GCGCCGCCGC 60
   GGCCGCGTCC CAGCAGCGGA GTAGGGCGGC GGCTGCGCCC CGCACCATGG GGGGCAGCCC 120
   AGCCCCAGCC GCGGTAAACG CCGACCTCCG CCGCCGCCCG CGCCGCGTCT GCCCCCTCCC 180
   GCTGCGGCTC TCTGGACGCC ATCCCCTCCT CACCTCGAAG CCAACATGAA GGAGACCCGG 240
   GGCTACGGAG GGGATGCCCC CTTCTGCACC CGCCTCAACC ACTCCTACAC AGGCATGTGG 300
   GCGCCCGAGC GTTCCGCCGA GGCGCGGGGC AACCTCACGC GCCCTCCAGG GTCTGGCGAG 360
   GATTGCGGAT CGGTGTCCGT GGCCTTCCCG ATCACCATGC TGCTCACTGG TTTCGTGGGC 420
   AACGCACTGG CCATGCTGCT CGTGTCGCGC AGCTACCGGC GCCGGGAGAG CAAGCGCAAG 480
   AAGTCCTTCC TGCTGTGCAT CGGCTGGCTG GCGCTCACCG ACCTGGTCGG GCAGCTTCTC 540
   ACCACCCCGG TCGTCATCGT CGTGTACCTG TCCAAGCAGC GTTGGGAGCA CATCGACCCG 600
   TCGGGGCGGC TCTGCACCTT TTTCGGGCTG ACCATGACTG TTTTCGGGCT CTCCTCGTTG 660
   TTCATCGCCA GCGCCATGGC CGTCGAGCGG GCGCTGGCCA TCAGGGCGCC GCACTGGTAT 720
   GCGAGCCACA TGAAGACGCG TGCCACCCGC GCTGTGCTGC TCGGCGTGTG GCTGGCCGTG 780
   CTCGCCTTCG CCCTGCTGCC GGTGCTGGGC GTGGGCCAGT ACACCGTCCA GTGGCCCGGG     840
   ACGTGGTGCT TCATCAGCAC CGGGCGAGGG GGCAACGGGA CTAGCTCTTC GCATAACTGG     900
   GGCAACCTTT TCTTCGCCTC TGCCTTTGCC TTCCTGGGGC TCTTGGCGCT GACAGTCACC     960
   TTTTCCTGCA ACCTGGCCAC CATTAAGGCC CTGGTGTCCC GCTGCCGGGC CAAGGCCACG    1020
   GCATCTCAGT CCAGTGCCCA GTGGGGCCGC ATCACGACCG AGACGGCCAT TCAGCTTATG    1080
   GGGATCATGT GCGTGCTGTC GGTCTGCTGG TCTCCGCTCC TGATAATGAT GTTGAAAATG    1140
   ATCTTCAATC AGACATCAGT TGAGCACTGC AAGACACACA CGGAGAAGCA GAAAGAATGC    1200
   AACTTCTTCT TAATAGCTGT TCGCCTGGCT TCACTGAACC AGATCTTGGA TCCTTGGGTT    1260
   TACCTGCTGT TAAGAAAGAT CCTTCTTCGA AAGTTTTGCC AGGAGGAATT TTGGGGAAAT    1320
   TAAAACCTGC CTTTCTGCCA GGATCACATC ACTGGAAGCT CCATGACTCT CTTTTTGTAA    1380
   AAGAAAA                                                              1387
(2) INFORMATION FOR SEQ ID NO:10:
   (i) SEQUENCE CHARACTERISTICS:
      (A) LENGTH: 390 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:10:
   Met Lys Glu Thr Arg Gly Tyr Gly Gly Asp Ala Pro Phe Cys Thr Arg
   1               5                   10                  15
   Leu Asn His Ser Tyr Thr Gly Met Trp Ala Pro Glu Arg Ser Ala Glu
               20                  25                  30
   Ala Arg Gly Asn Leu Thr Arg Pro Pro Gly Ser Gly Glu Asp Cys Gly
           35                  40                  45
   Ser Val Ser Val Ala Phe Pro Ile Thr Met Leu Leu Thr Gly Phe Val
       50                  55                  60
   Gly Asn Ala Leu Ala Met Leu Leu Val Ser Arg Ser Tyr Arg Arg Arg
   65                  70                  75                  80
   Glu Ser Lys Arg Lys Lys Ser Phe Leu Leu Cys Ile Gly Trp Leu Ala
                   85                  90                  95
   Leu Thr Asp Leu Val Gly Gln Leu Leu Thr Thr Pro Val Val Ile Val
               100                 105                 110
   Val Tyr Leu Ser Lys Gln Arg Trp Glu His Ile Asp Pro Ser Gly Arg
           115                 120                 125
   Leu Cys Thr Phe Phe Gly Leu Thr Met Thr Val Phe Gly Leu Ser Ser
       130                 135                 140
   Leu Phe Ile Ala Ser Ala Met Ala Val Glu Arg Ala Leu Ala Ile Arg
   145                 150                 155                 160
   Ala Pro His Trp Tyr Ala Ser His Met Lys Thr Arg Ala Thr Arg Ala
                   165                 170                 175
   Val Leu Leu Gly Val Trp Leu Ala Val Leu Ala Phe Ala Leu Leu Pro
               180                 185                 190
   Val Leu Gly Val Gly Gln Tyr Thr Val Gln Trp Pro Gly Thr Trp Cys
           195                 200                 205
   Phe Ile Ser Thr Gly Arg Gly Gly Asn Gly Thr Ser Ser Ser His Asn
       210                 215                 220
   Trp Gly Asn Leu Phe Phe Ala Ser Ala Phe Ala Phe Leu Gly Leu Leu
   225                 230                 235                 240
   Ala Leu Thr Val Thr Phe Ser Cys Asn Leu Ala Thr Ile Lys Ala Leu
                   245                 250                 255
   Val Ser Arg Cys Arg Ala Lys Ala Thr Ala Ser Gln Ser Ser Ala Gln
               260                 265                 270
   Trp Gly Arg Ile Thr Thr Glu Thr Ala Ile Gln Leu Met Gly Ile Met
           275                 280                 285
   Cys Val Leu Ser Val Cys Trp Ser Pro Leu Leu Ile Met Met Leu Lys
       290                 295                 300
   Met Ile Phe Asn Gln Thr Ser Val Glu His Cys Lys Thr His Thr Glu
   305                 310                 315                 320
   Lys Gln Lys Glu Cys Asn Phe Phe Leu Ile Ala Val Arg Leu Ala Ser
                   325                 330                 335
   Leu Asn Gln Ile Leu Asp Pro Trp Val Tyr Leu Leu Leu Arg Lys Ile
               340                 345                 350
   Leu Leu Arg Lys Phe Cys Gln Val Arg Tyr His Thr Asn Asn Tyr Ala
           355                 360                 365
   Ser Ser Ser Thr Ser Leu Pro Cys Gln Cys Ser Ser Thr Leu Met Trp
       370                 375                 380
   Ser Asp His Leu Glu Arg
   385                 390
(2) INFORMATION FOR SEQ ID NO:11:
   (i) SEQUENCE CHARACTERISTICS:
      (A) LENGTH: 388 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:11:
   Met Lys Glu Thr Arg Gly Tyr Gly Gly Asp Ala Pro Phe Cys Thr Arg
   1               5                   10                  15
   Leu Asn His Ser Tyr Thr Gly Met Trp Ala Pro Glu Arg Ser Ala Glu
               20                  25                  30
   Ala Arg Gly Asn Leu Thr Arg Pro Pro Gly Ser Gly Glu Asp Cys Gly
           35                  40                  45
   Ser Val Ser Val Ala Phe Pro Ile Thr Met Leu Leu Thr Gly Phe Val
       50                  55                  60
   Gly Asn Ala Leu Ala Met Leu Leu Val Ser Arg Ser Tyr Arg Arg Arg
   65                  70                  75                  80
   Glu Ser Lys Arg Lys Lys Ser Phe Leu Leu Cys Ile Gly Trp Leu Ala
                   85                  90                  95
   Leu Thr Asp Leu Val Gly Gln Leu Leu Thr Thr Pro Val Val Ile Val
               100                 105                 110
   Val Tyr Leu Ser Lys Gln Arg Trp Glu His Ile Asp Pro Ser Gly Arg
           115                 120                 125
   Leu Cys Thr Phe Phe Gly Leu Thr Met Thr Val Phe Gly Leu Ser Ser
       130                 135                 140
   Leu Phe Ile Ala Ser Ala Met Ala Val Glu Arg Ala Leu Ala Ile Arg
   145                 150                 155                 160
   Ala Pro His Trp Tyr Ala Ser His Met Lys Thr Arg Ala Thr Arg Ala
                   165                 170                 175
   Val Leu Leu Gly Val Trp Leu Ala Val Leu Ala Phe Ala Leu Leu Pro
               180                 185                 190
   Val Leu Gly Val Gly Gln Tyr Thr Val Gln Trp Pro Gly Thr Trp Cys
           195                 200                 205
   Phe Ile Ser Thr Gly Arg Gly Gly Asn Gly Thr Ser Ser Ser His Asn
       210                 215                 220
   Trp Gly Asn Leu Phe Phe Ala Ser Ala Phe Ala Phe Leu Gly Leu Leu
   225                 230                 235                 240
   Ala Leu Thr Val Thr Phe Ser Cys Asn Leu Ala Thr Ile Lys Ala Leu
                   245                 250                 255
   Val Ser Arg Cys Arg Ala Lys Ala Thr Ala Ser Gln Ser Ser Ala Gln
               260                 265                 270
   Trp Gly Arg Ile Thr Thr Glu Thr Ala Ile Gln Leu Met Gly Ile Met
           275                 280                 285
   Cys Val Leu Ser Val Cys Trp Ser Pro Leu Leu Ile Met Met Leu Lys
       290                 295                 300
   Met Ile Phe Asn Gln Thr Ser Val Glu His Cys Lys Thr His Thr Glu
   305                 310                 315                 320
   Lys Gln Lys Glu Cys Asn Phe Phe Leu Ile Ala Val Arg Leu Ala Ser
                   325                 330                 335
   Leu Asn Gln Ile Leu Asp Pro Trp Val Tyr Leu Leu Leu Arg Lys Ile
               340                 345                 350
   Leu Leu Arg Lys Phe Cys Gln Glu Ala Asn Ala Val Ser Ser Cys Ser
           355                 360                 365
   Asn Asp Gly Gln Lys Gly Gln Pro Ile Ser Leu Ser Asn Glu Ile Ile
       370                 375                 380
   Gln Thr Glu Ala
   385
(2) INFORMATION FOR SEQ ID NO:12:
   (i) SEQUENCE CHARACTERISTICS:
      (A) LENGTH: 365 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:12:
   Met Lys Glu Thr Arg Gly Tyr Gly Gly Asp Ala Pro Phe Cys Thr Arg
   1               5                   10                  15
   Leu Asn His Ser Tyr Thr Gly Met Trp Ala Pro Glu Arg Ser Ala Glu
               20                  25                  30
   Ala Arg Gly Asn Leu Thr Arg Pro Pro Gly Ser Gly Glu Asp Cys Gly
           35                  40                  45
   Ser Val Ser Val Ala Phe Pro Ile Thr Met Leu Leu Thr Gly Phe Val
       50                  55                  60
   Gly Asn Ala Leu Ala Met Leu Leu Val Ser Arg Ser Tyr Arg Arg Arg
   65                  70                  75                  80
   Glu Ser Lys Arg Lys Lys Ser Phe Leu Leu Cys Ile Gly Trp Leu Ala
                   85                  90                  95
   Leu Thr Asp Leu Val Gly Gln Leu Leu Thr Thr Pro Val Val Ile Val
               100                 105                 110
   Val Tyr Leu Ser Lys Gln Arg Trp Glu His Ile Asp Pro Ser Gly Arg
           115                 120                 125
   Leu Cys Thr Phe Phe Gly Leu Thr Met Thr Val Phe Gly Leu Ser Ser
       130                 135                 140
   Leu Phe Ile Ala Ser Ala Met Ala Val Glu Arg Ala Leu Ala Ile Arg
   145                 150                 155                 160
   Ala Pro His Trp Tyr Ala Ser His Met Lys Thr Arg Ala Thr Arg Ala
                   165                 170                 175
   Val Leu Leu Gly Val Trp Leu Ala Val Leu Ala Phe Ala Leu Leu Pro
               180                 185                 190
   Val Leu Gly Val Gly Gln Tyr Thr Val Gln Trp Pro Gly Thr Trp Cys
           195                 200                 205
   Phe Ile Ser Thr Gly Arg Gly Gly Asn Gly Thr Ser Ser Ser His Asn
       210                 215                 220
   Trp Gly Asn Leu Phe Phe Ala Ser Ala Phe Ala Phe Leu Gly Leu Leu
   225                 230                 235                 240
   Ala Leu Thr Val Thr Phe Ser Cys Asn Leu Ala Thr Ile Lys Ala Leu
                   245                 250                 255
   Val Ser Arg Cys Arg Ala Lys Ala Thr Ala Ser Gln Ser Ser Ala Gln
               260                 265                 270
   Trp Gly Arg Ile Thr Thr Glu Thr Ala Ile Gln Leu Met Gly Ile Met
           275                 280                 285
   Cys Val Leu Ser Val Cys Trp Ser Pro Leu Leu Ile Met Met Leu Lys
       290                 295                 300
   Met Ile Phe Asn Gln Thr Ser Val Glu His Cys Lys Thr His Thr Glu
   305                 310                 315                 320
   Lys Gln Lys Glu Cys Asn Phe Phe Leu Ile Ala Val Arg Leu Ala Ser
                   325                 330                 335
   Leu Asn Gln Ile Leu Asp Pro Trp Val Tyr Leu Leu Leu Arg Lys Ile
               340                 345                 350
   Leu Leu Arg Lys Phe Cys Gln Ile Glu Phe Trp Gly Asn
           355                 360                 365